# EUROPEAN PATENT APPLICATION

(11) **EP 4 840 471 A1**
(43) Date of publication of application: **18.11.2026**
(21) Application number: 25738541.9
(22) Date of filing: 10.01.2025
(51) Int. Cl.: A61K 47/68, A61K 47/65, A61K 31/4745, C07K 16/30, A61P 35/00

(54) **HYDROPHILIC ANTI-NECTIN-4 ANTIBODY-DRUG CONJUGATE, METHOD FOR PREPARING SAME, AND USE THEREOF**

(30) Priority: 12.01.2024 CN 202410051888
(71) Applicant: Hangzhou Adcoris Biopharma Co., Ltd, Hangzhou, Zhejiang 310052 (CN)
(72) Inventor: MIAO, Zhenwei, Hangzhou, Zhejiang 310052 (CN); HUANG, Yunsheng, Hangzhou, Zhejiang 310052 (CN)
(74) Representative: Elzaburu S.L.P.
(86) International application number: PCT/CN2025/071654
(87) International publication number: WO 2025/149013

(57) **Abstract**

Provided are a toxin-hydrophilic linker compound formed by camptothecin-7-ethylamine and a linker, a conjugate thereof with an anti-Nectin-4 antibody, and pharmaceutical use thereof. The toxin-hydrophilic linker compound can be conjugated with an anti-Nectin-4 monoclonal antibody to form an antibody-drug conjugate (ADC), so as to increase the hydrophilicity of the ADC, reduce the aggregation thereof in the circulatory system, improve the efficacy, and reduce the adverse effects.

## Description

The present application claims priority to Chinese Patent Application No. 2024100518885 filed on January 12, 2024, which is incorporated herein by reference in its entirety.

### TECHNICAL FIELD

The present disclosure relates to the field of medicine. Specifically, the present disclosure provides preparation of a series of camptothecin-7-ethylamine and toxin-hydrophilic linker compounds formed by the camptothecin-7-ethylamine and a linker, and an antibody conjugation formed by the camptothecin-7-ethylamine and an anti-Nectin-4 antibody, and their use in the treatment of tumors.

### BACKGROUND

Camptothecin and its derivatives have inhibitory activity against topoisomerase Top1, especially strong inhibitory activity against a Top1-DNA complex. Camptothecin has significant efficacy against gastric cancer, esophageal cancer, lung cancer, bladder cancer, etc., and is a broad-spectrum anti-tumor active drug. Camptothecin derivatives irinotecan and topotecan have been approved for the treatment of various cancers in many countries. Another camptothecin derivative, belotecan, has been approved for the treatment of small cell lung cancer (SCLC) and ovarian cancer in South Korea. The main drawbacks of camptothecin anti-tumor drugs are their toxicity, poor solubility, and the development of drug resistance by tumor cells.

### SUMMARY

The present disclosure provides a series of toxin-hydrophilic linker compounds formed by camptothecin-7-ethylamine and a linker, wherein the compound contains a hydrophilic structure consisting of at least two polyethylene glycols (PEGs). This toxin-hydrophilic linker can be conjugated with a monoclonal antibody Nectin-4 to form an antibody-drug conjugate (ADC), increasing the hydrophilicity of ADC, reducing the aggregation of ADC in the circulatory system, enhancing the efficacy, and reducing toxic side effects.

In one aspect, the present disclosure provides a compound of Formula (I), a pharmaceutically acceptable salt or a stereoisomer thereof: 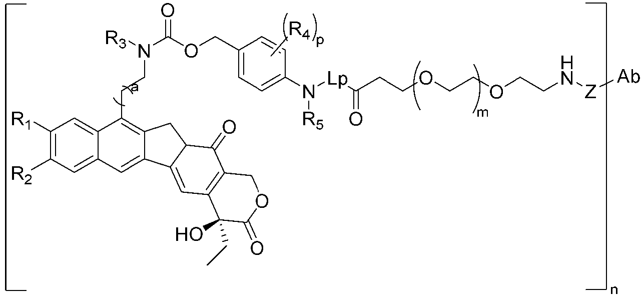 wherein,
R₁ and R₂ are each independently selected from hydrogen, fluorine, and C₁₋₃ alkyl, or R₁ and R₂ together with the carbon atoms to which they are attached form oxygen-containing heterocyclyl;
R₃ is selected from C₁₋₆ alkyl, C₁₋₆ alkoxy, C₃₋₆ cycloalkyl, or 4- to 8-membered heterocycloalkyl; the C₁₋₆ alkyl and the C₁₋₃ alkoxy are optionally substituted with one or more halogens; the 4- to 8-membered heterocycloalkyl contains 1, 2, or 3 heteroatoms selected from N, O, and S as ring atom;
R₄ is selected from hydrogen or heteroalkyl containing repeating units of - OCH₂CH₂-.
R₅ is selected from hydrogen, C₁₋₆ alkyl, and C₃₋₆ cycloalkyl;
Lp is selected from a peptide residue containing 1-5 amino acids;
m is selected from an integer ranging from 1 to 8;
a and p are selected from 1, 2, or 3;
Ab is an anti-Nectin-4 antibody or an antigen-binding fragment;
Z is a linker capable of conjugating the antibody or the antigen-binding fragment with other part of the compound of Formula (I);
$0.5 \leq n \leq 8 .$

In one embodiment, the compound of Formula (I) has the structure of Formula (I-1) or Formula (I-2): 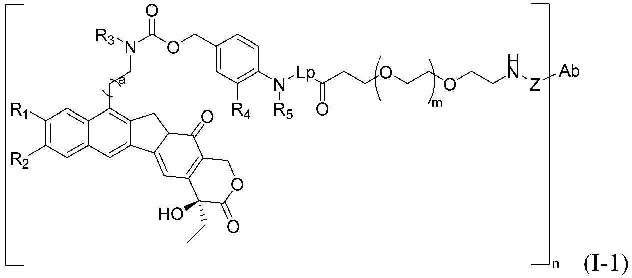 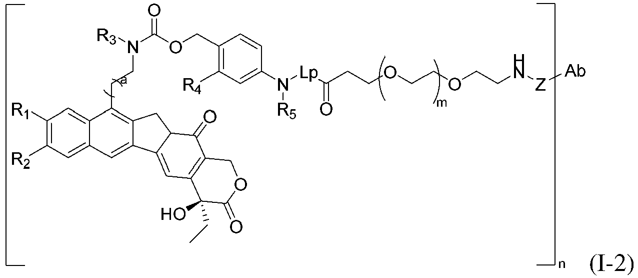 wherein the definition of each group is the same as that of the compound of Formula (I).

In one embodiment, m is selected from 1, 2, 3, 4, 5, 6, 7, or 8.

In one embodiment, R₁ and R₂ are each independently selected from hydrogen, fluorine, and methyl, or R₁ and R₂ together with the carbon atoms to which they are attached form

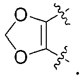

In one embodiment, R₁ is hydrogen, and R₂ is hydrogen.

In one embodiment, R₁ is fluorine, and R₂ is fluorine.

In one embodiment, R₁ is methyl, and R₂ is fluorine.

In one embodiment, R₃ is selected from C₁₋₆ alkyl substituted with 1, 2, or 3 fluorine.

In one embodiment, R₃ is selected from 4- to 8-membered oxacycloalkyl.

In one embodiment, R₃ is selected from oxetanyl, oxolanyl, or oxanyl.

In one embodiment, R₃ is selected from fluoroethyl, difluoroethyl, trifluoroethyl, oxolanyl, or oxanyl.

In one embodiment, R₄ is selected from hydrogen, C₁₋₆ alkyl, and -C(O)-NR^{a}R^{b}.
R^{a} and R^{b} are each independently selected from C₁₋₆ alkyl;
wherein one or more methylene units in the C₁₋₆ alkyl are optionally and independently replaced by -(OCH₂CH₂)q-;
q is selected from 2, 3, 4, 5, 6, 7, 8, 9 or 10.

In one embodiment, R₄ is selected from hydrogen,

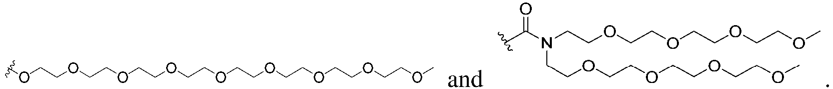

In one embodiment, L^{p} is selected from -Val-Cit-, -Gly-Lys-, -Gly-Leu-, -Val-Ala-, -Gly-Phe-, -Gly-Gly-Lys-, -Gly-Gly-Phe-, -Gly-Val-Ala-, -Gly-Gly-Val-, -Gly-Leu-Val-, -Gly-Phe-Gly-, or -Gly-Gly-Leu-.

In one embodiment, L^{p} is selected from

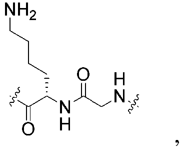

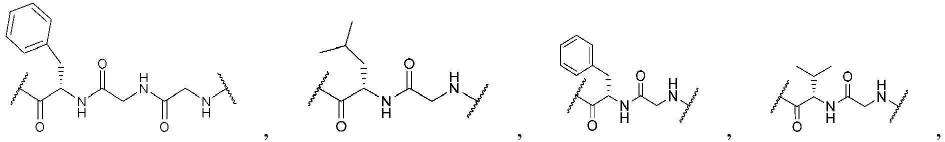

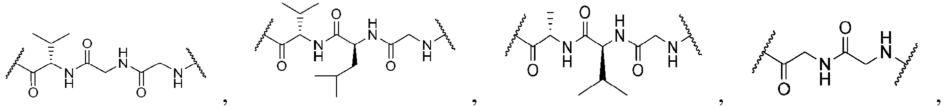

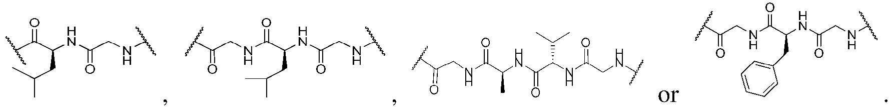

In the present disclosure, the linker for conjugating an antibody or antigen-binding fragment with other moieties of the compound of Formula (I) may be a single linker or a dual linker. The dual linker refers to a structure capable of simultaneously connecting two chemical functional groups (specifically, the antibody and a toxin moiety). The structure contains a group that is linked to the antibody and a group that is linked to the toxin moiety.

In one embodiment, Z is selected from 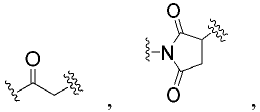 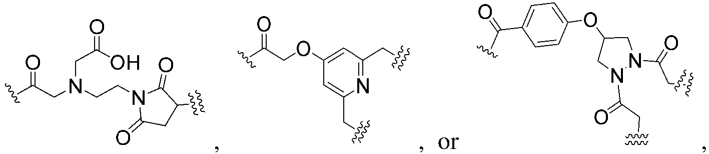 wherein the position indicated by 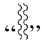 represents linking to an antibody, and the position indicated by 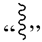 represents linking to -NH-.

In one embodiment, the heavy chain amino acid sequence of the anti-Nectin-4 antibody is as shown in SEQ ID NO: 1, and the light chain amino acid sequence is as shown in SEQ ID NO: 2.

The present disclosure provides the following toxin-linker compound, a pharmaceutically acceptable salt or a stereoisomer thereof:

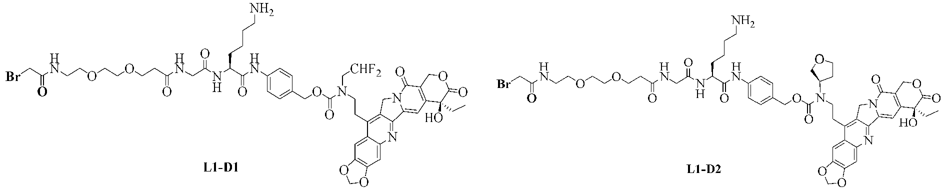

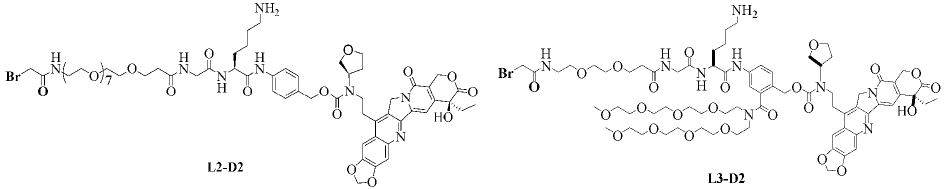

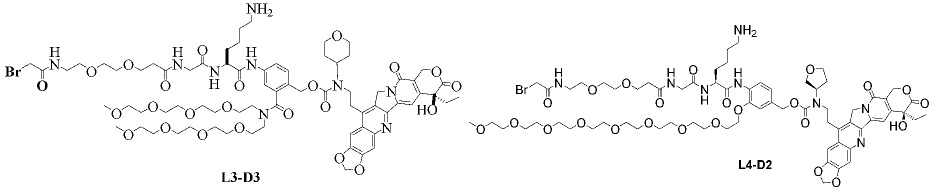

The present disclosure provides the following compound, a pharmaceutically acceptable salt or a stereoisomer thereof:

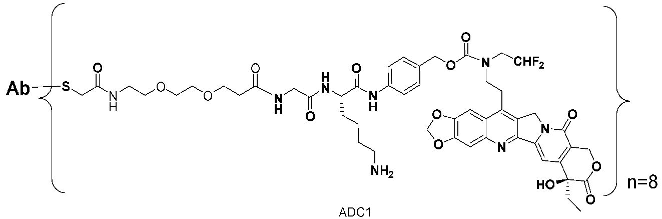

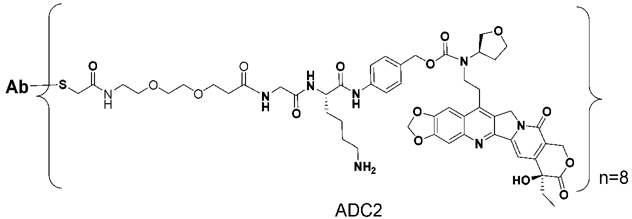

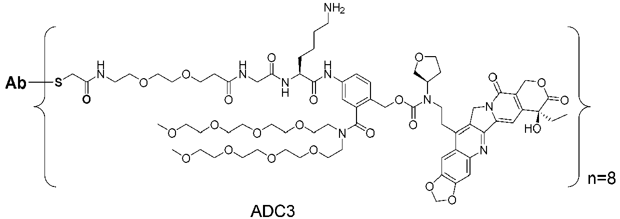

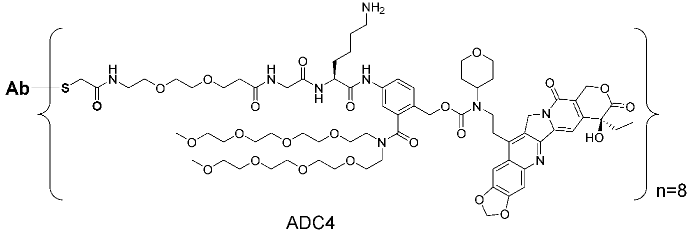

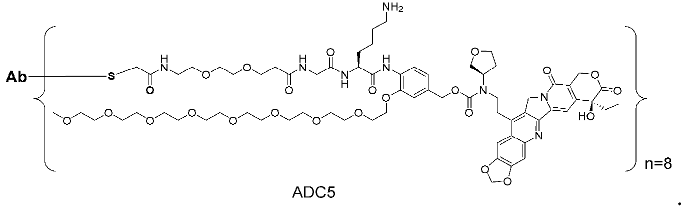

In another aspect, the present disclosure provides a pharmaceutical composition, comprising the aforementioned compound of Formula (I), the pharmaceutically acceptable salt or the stereoisomer thereof; and a pharmaceutically acceptable carrier.

In another aspect, the present disclosure provides use of the aforementioned compound of Formula (I), the pharmaceutically acceptable salt or the stereoisomer thereof, or the aforementioned pharmaceutical composition in the manufacture of an anti-tumor drug.

Preferably, the tumor is selected from solid tumor.

In one embodiment, the tumor is selected from breast cancer, bladder transitional cell carcinoma, prostate cancer, and pancreatic adenocarcinoma.

In another aspect, the present disclosure provides a method for treating cancer, comprising the step of administering to a patient in need thereof the aforementioned compound of Formula (I), the pharmaceutically acceptable salt or the stereoisomer thereof, or the aforementioned pharmaceutical composition.

In one embodiment, the cancer is selected from breast cancer, bladder transitional cell carcinoma, prostate cancer, and pancreatic adenocarcinoma.

In another aspect, the present disclosure provides a method for preparing a compound of Formula (I), comprising: 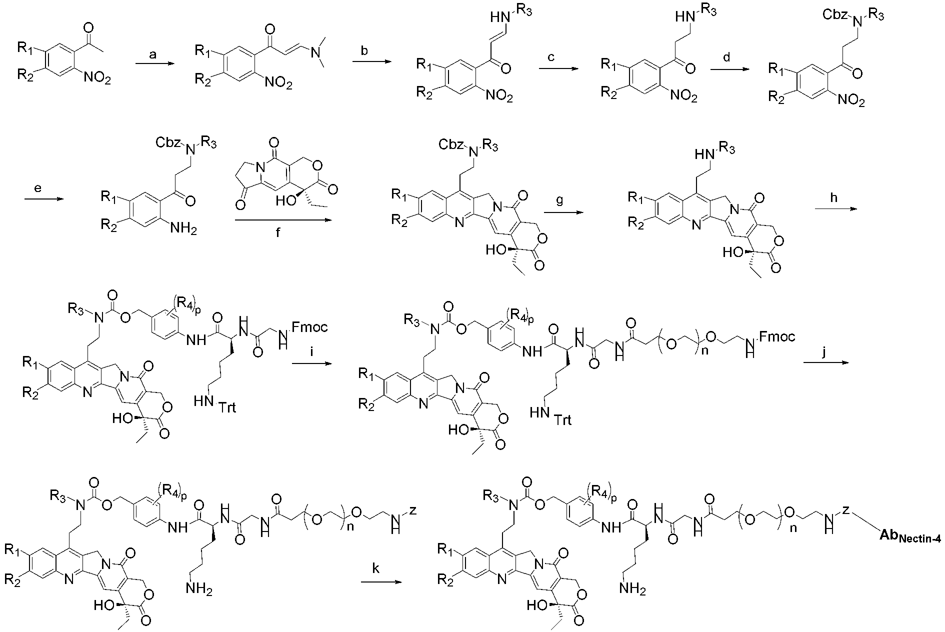 wherein each group is the same as defined in the compound of Formula (I);

The reagents for each step are adapted to those in Examples 1-5.

In one embodiment, the reagents used in each step are respectively: a) DMF-DMA; b) R₃-NH₂; c) NaH(OAc)₃; d) Cbz-Cl; e) Na₂S₂O₄; f) PPTS; g) H₂/Pd-C; h) Fmoc-Gly-Lys(Trt)-PABC; i) piperidine, followed by condensation with Fmoc-PEG2-OH; j) piperidine, followed by condensation with the corresponding Z linker; k) conjugation with anti-Nectin-4 antibody to form anti-Nectin-4 ADC.

### Beneficial Effects:

The toxin-hydrophilic linker and Nectin-4 monoclonal antibody provided by the present disclosure are conjugated to form antibody-drug conjugates (ADCs), which helps to reduce the aggregation of the ADCs in the circulatory system, improve drug efficacy, and reduce toxic side effects.

### BRIEF DESCRIPTION OF THE DRAWINGS

FIG. 1 shows reversed-phase liquid chromatography-mass spectrometry (RP-MS) test results of ADC1 in Example 1; wherein A and B are high performance liquid chromatography (HPLC) results, and C and D are MS results.
FIG. 2 shows RP-MS test results of ADC2 in Example 2; wherein A and B are HPLC results, and C and D are MS results.
FIG. 3 shows RP-MS test results of ADC3 in Example 3; wherein A and B are HPLC results, and C and D are MS results.
FIG. 4 shows RP-MS test results of ADC4 in Example 4; wherein A and B are HPLC results, and C and D are MS results.
FIG. 5 shows RP-MS test results of ADC5 in Example 5; wherein A and B are HPLC results, and C and D are MS results.

### DETAILED DESCRIPTION

### I. Definition

In the present disclosure, unless otherwise indicated, scientific and technical terms used herein have the meanings commonly understood by those skilled in the art. Moreover, the relevant terminology and laboratory procedures used herein are those widely used and conventional in the respective fields. Meanwhile, for a better understanding of the present disclosure, definitions and explanations of relevant terms are provided below.

As used herein and unless otherwise specified, the term "about" or "approximately" means within plus or minus 10% of a given value or range. Where an integer is required, the term means within plus or minus 10% of a given value or range, rounded up or down to the nearest integer.

In the description herein, references to "some embodiments", "some implementations", or "some aspects" describe a subset of all possible embodiments. However, it should be understood that "some embodiments" may refer to the same subset or different subsets of all possible embodiments, and may be combined with each other without conflict.

As used herein and unless otherwise specified, the terms "comprise", "include", "have", and "contain", including their grammatical equivalents thereof, are generally to be understood as open-ended and non-limiting, e.g., not excluding other unlisted elements or steps.

The term "antibody" as used in this application is employed in the broadest sense and specifically encompasses monoclonal antibodies, polyclonal antibodies, dimers, multimers, multispecific antibodies (e.g., bispecific antibodies), and antibody fragments, provided that they exhibit the desired biological activity. Antibodies can be murine, human, humanized, chimeric, or derived from other species. Antibodies are proteins generated by the immune system that can recognize and bind to specific antigens. Target antigens typically possess multiple binding sites, also known as epitopes, which are recognized by complementarity-determining regions (CDRs) on various antibodies. Each antibody that specifically binds to distinct epitopes has a distinct structure. Therefore, an antigen can have more than one corresponding antibody. Antibody comprises full-length immunoglobulin molecules or immunologically active portions of full-length immunoglobulin molecules, i.e., molecules comprising an antigen-binding site that immunospecifically binds to a target antigen or a portion thereof, these targets including but not limited to cancer cells or cells producing autoantibodies associated with autoimmune diseases. The term "antibody" refers to an immunoglobulin molecule capable of specifically binding to an antigen. It comprises two light chains with relatively low molecular weight and two heavy chains with relatively high molecular weight, with the heavy chains (H chains) and light chains (L chains) linked by disulfide bonds to form a tetrapeptide chain molecule.

The term "antibody-drug conjugate (ADC)" as used in this application refers to a biologically active small molecule drug linked to a monoclonal antibody via a chemical linker, with the monoclonal antibody serving as a carrier to deliver the small molecule drug to target cells.

The term "toxin" used in this application is also referred to as "cytotoxic drug moiety" or "small molecule drug" which refers to a compound that exerts a killing effect on tumor cells. Examples of the cytotoxic drug moiety include at least one of anti-microtubule agents, DNA intercalating agents, DNA topoisomerase inhibitors, DNA synthesis inhibitors, RNA polymerase inhibitors, spliceosome inhibitors, proteolysis-targeting chimeras (PROTACs), and immunomodulators.

The small molecule drugs used in this application can be asymmetric, for example, having one or more stereoisomers. Unless otherwise specified, all stereoisomers, such as enantiomers and diastereomers, are included. The stereoisomers include geometric isomers (e.g., cis/trans configurations) and optical isomers (e.g., enantiomers), as therapeutics composed of monomers, racemates, racemic mixtures, and pharmaceutically acceptable salts thereof. The compounds of the present application containing asymmetric carbon atoms can be isolated in an optically active pure form or in a racemic form. Optically pure forms can be resolved from racemic mixtures or synthesized using chiral starting materials or chiral reagents. Racemates, diastereomers, and enantiomers are all included within the scope of this application.

The small molecule drugs used in this application also include tautomeric forms. Tautomeric forms arise from the exchange of a single bond with an adjacent double bond, accompanied by the migration of a proton.

In this document, the number range refers to each integer within a given range. For example, "C₁-C₆" indicates that the group may have 1 carbon atom, 2 carbon atoms, 3 carbon atoms, 4 carbon atoms, 5 carbon atoms, or 6 carbon atoms; "C₃-C₆" indicates that the group may have 3 carbon atoms, 4 carbon atoms, 5 carbon atoms, or 6 carbon atoms.

When any variable (e.g., Rₙ) appears more than once in the composition or structure of a compound, its definition in each instance is independent. Thus, for example, if a group is substituted with 1-5 R, the group may optionally be substituted with at most 5 R, and each occurrence of R is independently selected. In addition, combinations of substituents and/or variants thereof are permitted only if such combinations result in stable compounds.

The term "alkyl" as used in this application refers to a saturated aliphatic hydrocarbon group, which is a straight or branched chain group containing 1 to 20 carbon atoms, preferably an alkyl group containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, and 2,3-dimethylbutyl. Alkyl may be substituted or unsubstituted. When substituted, the substituent(s) can be attached at any available point of attachment, and are preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate ester. In the present application, methyl, ethyl, isopropyl, tert-butyl, haloalkyl, deuterated alkyl, alkoxy-substituted alkyl, and hydroxy-substituted alkyl are preferred.

The term "heterocyclyl" or "heterocycloalkyl" used in this application refers to a saturated monocyclic or polycyclic cyclic hydrocarbon substituent containing 3 to 20 ring atoms, wherein one or more ring atoms are heteroatoms selected from nitrogen, oxygen, or S(O)ₘ (where m is an integer from 0 to 2), but excludes ring moieties of -O-O-, -O-S-, or -S-S-, and the remaining ring atoms are carbon.

The term "alkoxy" as used in this application refers to -O-(alkyl) and -O-(unsubstituted cycloalkyl), wherein alkyl is the same as defined above. Non-limiting examples of alkoxy groups include: methoxy, ethoxy, propoxy, butoxy, cyclopropoxy, cyclobutoxy, cyclopentoxy, cyclohexoxy.

The term "substituted" as used in this application means that one or more hydrogen atoms in a group, preferably up to 5, more preferably 1 to 3 hydrogen atoms, are independently substituted by the corresponding number of substituents. It goes without saying that the substituents are only in their possible chemical positions, and a person skilled in the art can determine, without undue effort (by experiment or theory), the possible or impossible substitutions. For example, an amino group or hydroxyl group having a free hydrogen atom may be unstable when bonded to a carbon atom having an unsaturated (e.g., olefinic) bond. 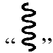 refers to the point of chemical bond connection.

The term "pharmaceutically acceptable salt" used in this application refers to a salt formed by the corresponding amine compound with an inorganic or organic acid, or a salt formed by the corresponding carboxylic acid compound with an alkali metal or alkaline earth metal, or a salt formed by the corresponding carboxylic acid compound with an organic amine. Among them, inorganic acids include but are not limited to hydrochloric acid, hydrobromic acid, hydroiodic acid, sulfuric acid, phosphoric acid, etc.; organic acids include but are not limited to acetic acid, propionic acid, butyric acid, benzoic acid, methanesulfonic acid, benzenesulfonic acid, p-toluenesulfonic acid, oxalic acid, succinic acid, lactic acid, citric acid, succinic acid, gluconic acid, maleic acid, fumaric acid, tartaric acid, etc.; alkali metal or alkaline earth metal salts include but are not limited to sodium, potassium, calcium, magnesium salts, etc.; organic amine salts include but are not limited to salts composed of ammonia, methylamine, ethylamine, propylamine, isopropylamine, dimethylamine, diethylamine, trimethylamine, triethylamine, tert-butylamine, ethylenediamine, ethanolamine, diethanolamine, triethanolamine, morpholine, piperidine, piperazine, amino acids, etc.

The drug or pharmaceutical composition of the present application can be administered orally, topically, parenterally, or mucosally (e.g., buccally, by inhalation, or rectally) in dosage unit formulations containing conventional non-toxic pharmaceutically acceptable carriers. The oral route is generally preferred. The active agent may be administered orally in the form of capsules, tablets, etc. (see Remington: The Science and Practice of Pharmacy, 20th Edition).

For oral administration in the form of tablets or capsules, the active pharmaceutical ingredient may be combined with non-toxic, pharmaceutically acceptable excipients such as binders (e.g., pregelatinized corn starch, polyvinylpyrrolidone, or hydroxypropyl methylcellulose); fillers (e.g., lactose, sucrose, glucose, mannitol, sorbitol, and other reducing and non-reducing sugars, microcrystalline cellulose, calcium sulfate, or dibasic calcium phosphate); lubricants (e.g., magnesium stearate, talc or silica, stearic acid, sodium stearyl fumarate, glyceryl behenate, calcium stearate, etc.); disintegrants (e.g., potato starch or sodium starch glycolate); or wetting agents (e.g., sodium lauryl sulfate), colorants and flavoring agents, gelatin, sweeteners, natural and synthetic gums (such as acacia, tragacanth, or alginates), buffering salts, carboxymethylcellulose, polyethylene glycols, waxes, and the like. For oral administration in liquid form, the pharmaceutical composition can be combined with non-toxic, pharmaceutically acceptable inert carriers (e.g., ethanol, glycerol, water), suspending agents (e.g., sorbitol syrup, cellulose derivatives, or hydrogenated edible fats), emulsifying agents (e.g., lecithin or acacia), non-aqueous carriers (e.g., almond oil, oil esters, ethanol, or fractionated vegetable oils), preservatives (e.g., methyl p-hydroxybenzoate or propyl p-hydroxybenzoate or sorbic acid), and the like. Stabilizers such as antioxidants (BHA, BHT, propyl gallate, sodium ascorbate, citric acid) can also be added to stabilize the dosage form. Tablets comprising an active compound can be coated by methods well known in the art. The composition of the present application, which comprises a compound of Formula I as an active compound, may further incorporate beads, microspheres, or microcapsules, for example those constructed from polyglycolic acid/lactic acid (PGLA). Liquid preparations for oral administration may take the form of, for example, solutions, syrups, emulsions, or suspensions, or they may be presented as dry products for reconstitution with water or other suitable vehicles prior to use. Formulations for oral administration can be suitably prepared to achieve controlled or delayed release of the active compound.

The term "treatment" used in this application includes inhibiting, alleviating, preventing, or eliminating one or more symptoms or side effects associated with the disease, condition, or disorder being treated.

The use of the term "inhibition" in this application is relative to a control. A person skilled in the art will readily determine appropriate controls for each experiment. For example, the reduced response in a subject or cell treated with a compound is compared with the response in a subject or cell not treated with the compound.

The term "pharmaceutical composition" as used in this application refers to a composition comprising a compound described herein or a pharmaceutically acceptable salt thereof, and at least one pharmaceutically acceptable ingredient selected from the following depending on the mode of administration and the nature of the dosage form, including but not limited to: carriers, diluents, adjuvants, excipients, preservatives, fillers, disintegrants, wetting agents, emulsifiers, suspending agents, sweeteners, flavoring agents, fragrances, antibacterial agents, antifungal agents, lubricants, dispersants, temperature-sensitive materials, temperature regulators, adhesives, stabilizers, suspending aids, and the like.

### II. Examples

In order to make the objectives, technical solutions, and advantages of the present disclosure clearer, the present disclosure will be described in further detail below. The described embodiments should not be construed as limiting the present disclosure, and all other embodiments obtained by those of ordinary skill in the art without creative effort shall fall within the scope of protection of the present disclosure.

Before providing further detailed description of the embodiments of the present disclosure, the nouns and terms involved in the embodiments of the present disclosure are explained, and the nouns and terms involved in the embodiments of the present disclosure are applicable to the following interpretations.

The raw materials and equipment used in the specific embodiments of the present disclosure are all known products, obtained by purchasing commercially available products.
DAR value testing and calculation: Based on the RP-HPLC-MS test results, the DAR value of the ADC was analyzed using a Waters Acquity UPLC I-Class/Xevo G2-XS QTOF instrument.

RP-HPLC parameter settings: column PLRP-S 1000A 5UM, column temperature 70°C. Mobile phase A is0.1% formic acid in water, mobile phase B is 0.1% formic acid in acetonitrile, flow rate is 0.2 mL/min. The mobile phase gradient is 20-50% B over 18 minutes; 50-95% B over 5 minutes; 95-20% B over 0.1 minute; and 20-20% B over 6.9 minutes.

MS parameter settings: capillary voltage 2.50 kV, cone voltage 100 V, mass analysis range m/z 200 to 4000, MSE collision energy 20 to 45 eV, ion source temperature 120°C, desolvation temperature 500°C, desolvation gas flow 1000 L/Hr, internal standard: leucine enkephalin. The test sample is diluted to 1 mg/mL using sample buffer. TCEP is added to reach a final concentration of 50 mmol/L. The mixture is incubated at 37°C for 20 min, followed by injection of 5 µL of the resulting solution. The light chain peak is identified, and its peak area percentage is calculated: with the total peak area normalized to 100. Likewise, the heavy chain peak is identified, and its peak area percentage is calculated: with the total peak area normalized to 100. The weighted peak areas of the heavy chain and the light chain are calculated: by multiplying the peak area percentage by the corresponding drug load, respectively. The formula for calculating the DAR value is as follows: DAR = 2 * (Σ weighted peak area of light chains + Σ weighted peak area of heavy chains)/100.

In this example, ADC is prepared by using a Nectin-4 antibody, but not limited thereto.

The amino acid sequence of the heavy chain of the Nectin-4 antibody is as follows (SEQ ID NO: 1):

EVQLVESGGGLVQPGGSLRLSCAASGFTFSSYNMNWVRQAPGKGLEW
VSYISSSSSTIYYADSVKGRFTISRDNAKNSLSLQMNSLRDEDTAVYYCARAYYYGMD
VWGQGTTVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKRVEPKSCD

KTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKT
ISKAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

The amino acid sequence of the light chain of the Nectin-4 antibody is as follows (SEQ ID NO: 2):

DIQMTQSPSSVSASVGDRVTITCRASQGISGWLAWYQQKPGKAPKFLI
YAASTLQSGVPSRFSGSGSGTDFTLTISSLQPEDFATYYCQQANSFPPTFGGGTKVEIK
RTVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

### Example 1: Ab_{nectin-4}-S-(7-(N-(acetamide-PEG₂-propionyl-Gly-Lys-PABC)-N-(2'2'-difluoroethyl))aminoethyl camptothecin)₈ (ADC1)

### 1.1 7-(N-(bromoacetamide-PEG₂-propionyl-Gly-Lys-PABC)-N-(2'2'-difluoroethyl))aminoethyl camptothecin (L1-D1)

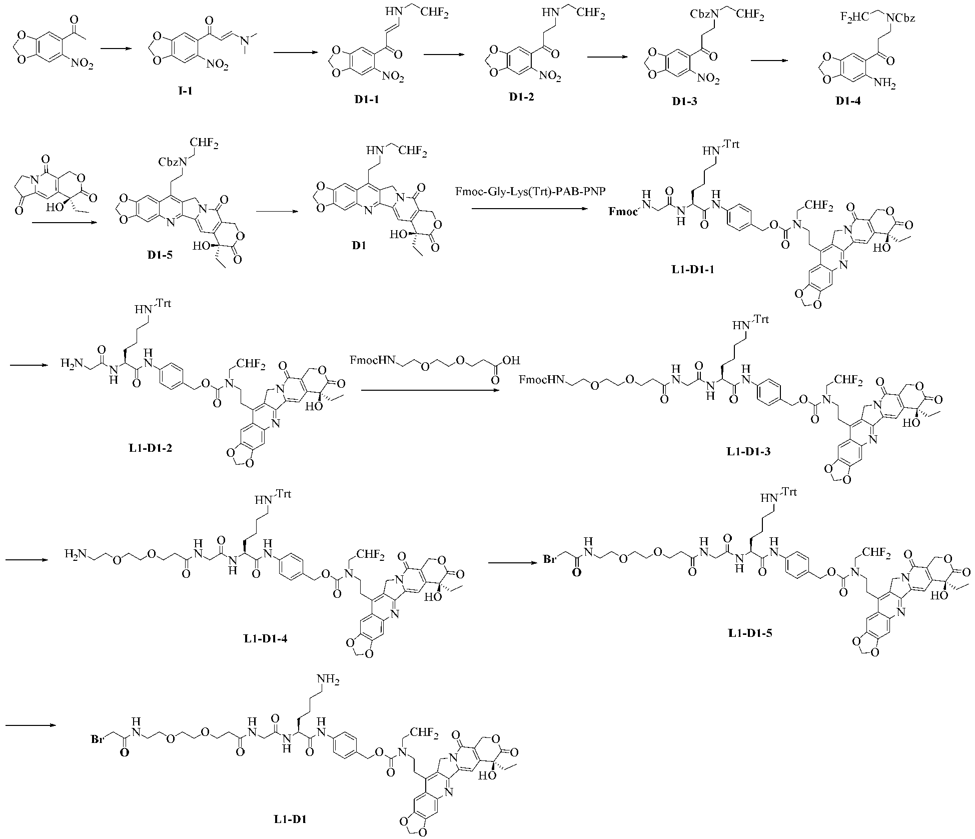

4',5'-methylenedioxy-2'-nitroacetophenone (15 g, 71.72 mmol) was added to a 500 mL single-neck flask, and DMF-DMA (170.92 g, 1.43 mol) was added. the mixture was heated to 100°C and the mixture was heated under reflux for 10 h. The reaction product was concentrated to obtain a yellow solid. THF (30 mL) was added, and n-hexane (150 mL) was dropwise added. The resulting mixture was stirred for 3 h and then filtered to obtain a wet yellow solid. The obtained wet yellow solid was dried in vacuum at 40°C for 6 h to obtain a light yellow solid intermediate **I-1** (18 g, yield 95%, HPLC 98%); LCMS: [M+1]⁺265.22 (calculated: 264.24); ¹H NMR (600 MHz, DMSO-d₆) δ 7.62-7.22 (m, 2H), 7.04 (s, 1H), 6.24 (s, 2H), 5.22 (d,*J*=6.9 Hz, 1H), 3.08 (s, 3H), 2.84 (s, 3H).

The intermediate **I-1** (18 g, 68.12 mmol) was added to IPA (180 mL), the reaction solution was stirred, DIEA (17.61 g, 136.24 mmol) and difluoroethylamine hydrochloride (32.02 g, 272.48 mmol) were added. The resulting mixture was refluxed under stirring to react for 12 h. The reaction product was concentrated, water (200 mL) was added, and the mixture was stired for 2 h and filtered to obtain a yellow solid. The obtained yellow solid was dried at 40°C to obtain a product **D1-1** (15.8 g, yield 77.3%, HPLC 98%); LCMS: [M+1]⁺301.47 (calculated: 300.22); ¹H NMR (600 MHz, CDCl₃) δ 9.84 (s, 1H), 7.40 (s, 1H), 6.87 (s, 1H), 6.84 (dd,*J*=12.6, 7.5 Hz, 1H), 6.13 (s, 2H), 5.99-5.74 (m, 1H), 5.26 (d,*J*=7.5 Hz, 1H), 3.68-3.48 (m, 2H).

The compound **D1-1** (15.8 g, 52.63 mmol) was added to DCM (310 mL), and then acetic acid (63.05 g, 1.05 mol) was added. The solids were fully dissolved under stirring. The internal temperature of the reaction solution was maintained at 15°C. Sodium triacetoxyborohydride (27.89 g, 131.56 mmol) was added in 4 batches, approximately every 5 min, about 7 g each time. The internal temperature of the reaction solution was maintained at 15°C to react for 2 h. The reaction solution was poured into a 0°C sodium carbonate aqueous solution (sodium carbonate: 130 g, water: 1 L), the internal temperature was maintained at 15°C. The reaction solution was stirred, and the layers were separated. The organic phase was washed with water. The washed organic phase was then collected and dried over anhydrous sodium sulfate to obtain compound **D1-2** (used directly in the next step). The obtained compound was cooled to 0°C, DIEA (13.58 g, 105.26 mmol) was added, a solution of CbzCl (10.11 g, 59.27 mmol) in DCM was added dropwise (dropwise addition was completed in about 20 min). The mixture reacted at 0°C for 12 h, then the reaction product was added to a10% citric acid aqueous solution (200 mL * 2), washed twice, and then washed successively with saturated sodium bicarbonate aqueous solution (200 mL) and saturated brine (200 mL). The organic phase was collected and dried over anhydrous sodium sulfate, and concentrated to obtain product **D1-3** (19.7 g, 85.7% overall yield over two steps, 95% purity by HPLC); LCMS: [M+1]⁺437.42 (calculated: 436.37); ¹H NMR (500 MHz, CDCl₃) δ 7.40-7.28 (m, 7H), 6.17 (s, 2H), 5.15 (d,*J*=4.5 Hz, 2H), 3.82-3.65 (m, 4H), 3.14-2.75 (m, 3H).

Under nitrogen protection, compound **D1-3** (19.7 g, 45.1 mmol) was added to a 1 L single-neck flask, followed by the addition of DMF (50 mL). The mixture was cooled to 0°C, and a pre-prepared reducing solution consisting of water (250 mL), sodium hydrosulfite (39.30 g, 225.72 mmol) and sodium carbonate (19.14 g, 180.58 mmol) was added slowly. Upon completion of the addition, the reaction system was gradually heated to 40°C and stirred for 2 h. The mixture was then filtered, and the filtrate was concentrated. Ethyl acetate (500 mL) and water (500 mL) were added for extraction and phase separation. Ethyl acetate (500 mL) was added to the aqueous phase, and the pH value was adjusted to 3-4 with 2 M dilute hydrochloric acid. The resulting mixture was stirred at room temperature for 6 h before extraction and phase separation. The organic phases were combined and dried over sodium sulfate. After concentration, the crude product was purified via silica gel column chromatography to obtain yellow oil **D1-4.** (17.7 g, yield 96.7%, HPLC 98%); LCMS: [M+1]⁺407.52 (calculated: 406.39); ¹H NMR (500 MHz, CDCl₃) δ 7.44-7.02 (m, 7H), 6.03 (s, 2H), 5.79-5.72 (m, 2H), 5.10-4.98 (m, 2H), 3.73-3.48 (m, 3H), 3.04 (m, 2H), 2.97-2.88 (m, 2H).

Compound **D1-4** (17.7 g, 43.55 mmol), (S)-4-ethyl-4-hydroxy-7,8-dihydro-1H-pyrano[3,4-f]indolizine-3,6,10(4H)-trione (13.75 g, 52.27 mmol) and PPTS (218.88 g, 871.00 mmol) were successively added to a 500 mL single-neck flask. The mixture was stirred and heated to 110°C for 12 h under argon protection. The reaction solution was poured into methanol (1000 mL) and stirred for 2 h, and then a dark brown solid was collected by filtration. The brown solid was stirred in methanol (300 mL) for another 2 h and filtered again. The resulting light brown solid was dried at 40°C to obtain compound **D1-5**. (10.1 g, yield 36.6%, HPLC 96%); LCMS: [M+1]⁺634.33 (calculated: 633.60); ¹H NMR (600 MHz, DMSO-*d*₆) δ 7.47-7.17 (m, 7H), 7.17 (d,*J*=7.7 Hz, 1H), 6.48 (s, 1H), 6.24 (d,*J*=6.2 Hz, 2H), 6.13 (dq,*J*=55.7, 3.8 Hz, 1H), 5.52-5.26 (m, 2H), 5.15 (s, 1H), 5.11-4.83 (m, 3H), 3.93-3.68 (m, 2H), 3.53 (dt,*J*=42.0, 7.9 Hz, 2H), 3.22 (t,*J*=8.2 Hz, 2H), 1.89 (ddp, *J*=21.1, 14.0, 6.9, 6.0 Hz, 2H), 0.90 (q,*J*=6.8 Hz, 3H).

Compound **D1-5** (10 g, 15.78 mmol), dichloromethane (2.5 L) and methanol (2.5 L) were successively added to a 10 L single-neck flask. 10% Pd/C (15 g) was added to the reaction solution with stirring. The mixture was reacted at room temperature under a hydrogen balloon atmosphere for 20 h. Pd/C was removed by filtration, and the reaction solution was concentrated to dryness. The residue was stirred with 30 mL of dichloromethane-methanol mixed solution (V/V = 1:1) for 24 h, followed by filtration. The filtrate was dried at 40°C for 3 h to obtain product **D1**. (5.8 g, yield 73.6%, HPLC 95.0%); LCMS: [M+1]⁺500.52 (calculated: 499.47); ¹H NMR (500 MHz, DMSO-d₆) δ 7.68 (s, 1H), 7.52 (s, 1H), 7.28 (s, 1H), 6.58 (s, 1H), 6.33 (s, 2H), 6.02 (t,*J*=56.6 Hz, 1H), 5.45-5.45 (m, 2H), 5.45-5.45 (m, 2H), 3.35-3.25 (m, 2H), 3.05-3.85 (m, 4H), 1.85-1.95 (d,*J*=58.5 Hz, 2H), 1.05-0.85 (m, 3H).

Preparation of **Fmoc-Gly-Lys(Trt)-PAB-PNP**: In a 1 L single-neck flask, Fmoc-Lys(Trt) (50 g, 82.1 mmol) and 4-aminobenzyl alcohol (15.1 g, 122.7 mmol) were dissolved in a mixed solvent of DCM (250 mL) and MeOH (250 mL). The solution was cooled to 0°C, and 2-ethoxy-1-ethoxycarbonyl-1,2-dihydroquinoline (EEDQ) (30.3 g, 122.7 mmol) was added in portions. After being held at the temperature for 10 min, the mixture reacted at room temperature overnight. The reaction solution was washed with a 2% aqueous citric acid solution (300 mL), followed by a saturated sodium bicarbonate aqueous solution (300 mL). The organic phase was washed with a saturated brine, dried over anhydrous sodium sulfate and then concentrated to obtain **Fmoc-Lys(Trt)-PAB** as an oily product. (LCMS: [M+1]⁺716.30 (calculated: 715.35).

Dichloromethane (DCM) (300 mL) was added to the above oily product. 8-diazabicyclo[5.4.0]undec-7-ene (DBU) (7.4 g, 48.9 mmol) was added with stirring, and the above mixture reacted at room temperature for 2 h. The reaction solution was concentrated to approximately 200 mL and then added dropwise into 2 L of methyl tert-butyl ether. The resulting reaction mixture was filtered, the filter cake was evaporated to be dryness at room temperature to obtain **Lys(Trt)-PAB** as a pink solid. (37 g, yield 91% over two steps); LCMS: [M+1]⁺494.70 (calculated: 493.65).

Lys(Trt)-PAB (47 g, 95.2 mmol) was added in a 1 L single-neck flask and dissolved in DCM (500 mL). Fmoc-glycine (11.3 g, 38.1 mmol), 1-ethyl-3-(3-dimethylaminopropyl)carbodiimide hydrochloride (EDCI) (18.2 g, 95.2 mmol), 1-hydroxybenzotriazole (HOBT) (12.8 g, 95.2 mmol) and pyridine (7.5 g, 95.2 mmol) were added sequentially. The mixture was stirred at room temperature for 2 h. The reaction solution was washed with 300 mL of 5% citric acid aqueous solution, followed by 200 mL of saturated sodium bicarbonate aqueous solution. The organic phase was washed with saturated brine, dried over anhydrous sodium sulfate, and concentrated to obtain **Fmoc-Gly-Lys(Trt)-PAB**. (40 g, yield 54%); LCMS: [M+1]⁺773.70 (calculated: 772.95); ¹H NMR (600 MHz, DMSO-d₆) δ 10.00 (s, 1H), 8.12 (d,*J*=7.5 Hz, 1H), 7.95-7.86 (m, 2H), 7.74 (d,*J*=7.4 Hz, 2H), 7.68-7.57 (m, 3H), 7.53-7.14 (m, 20H), 5.16 (t,*J*=5.3 Hz, 1H), 4.47 (dd,*J*=16.1, 5.9 Hz, 3H), 4.28 (dd,*J*=24.2, 6.7 Hz, 3H), 3.72 (s, 2H), 2.52 (d,*J*=7.4 Hz, 1H), 1.97 (s, 2H), 1.80-1.20 (m, 6H).

**Fmoc-Gly-Lys(Trt)-PAB** (40 g, 51.7 mmol) and DCM (400 mL) were successively added into a 1 L single-neck flask and stirred until fully dissolved. TEA (15.6 g, 155.1 mmol) and bis(4-nitrophenyl) carbonate (47.1 g, 155.1 mmol) were added dropwise at room temperature. The mixture was stirred at room temperature for 2 h, and extracted with water (400 mL). The organic phase was washed with saturated brine, dried over anhydrous sodium sulfate and evaporated to dryness, and the crude product was purified by silica gel column chromatography to obtain **Fmoc-Gly-Lys(Trt)-PAB-PNP**. (17.5 g, yield 36%, HPLC 95.7%); LCMS:[M+1]⁺+ 938.62 (calculated: 937.37); ¹H NMR (500 MHz, DMSO-d₆)δ 10.16 (s, 1H), 8.35 (d,*J*=9.1 Hz, 2H), 8.20-8.10 (m, 2H), 7.92 (d,*J*=7.5 Hz, 2H), 7.80-7.55 (m, 7H), 7.50-7.14 (m, 20H), 5.29 (s, 2H), 4.50-4.40 (m, 1H), 4.34-4.22 (m, 3H), 3.77-3.68 (m, 2H), 2.00-1.85 (m, 2H), 1.79-1.25 (m, 7H).

**D1** (3.7 g, 7.41 mmol), N-methyl-2-Pyrrolidone (NMP) (37 mL), N,N-diisopropylethylamine (DIPEA) (2.87 g, 22.22 mmol), Fmoc-Gly-Lys(Trt)-PAB-PNP (6.95 g, 7.41 mmol) and HOBT (1.05 g, 7.77 mmol) were successively added to a 50 mL single-neck flask. The mixture was stirred at room temperature for 2 h. The reaction solution was washed with 50 mL of 2% citric acid aqueous solution, followed by 50 mL of saturated sodium bicarbonate aqueous solution. The organic phase was washed with 50 mL of saturated brine, dried over anhydrous sodium sulfate and concentrated to obtain crude product **L1-D1-1** which was used directly for the next-step reaction; LCMS: [M+1]⁺ 1298.74 (calculated: 1297.50); ¹H NMR (600 MHz, DMSO-*d*₆) δ 10.06 (d, *J*=23.4 Hz, 1H), 8.11 (d, *J*=7.8 Hz, 1H), 7.86 (d, *J*=7.7 Hz, 1H), 7.93-7.47 (m, 6H), 7.62-7.27 (m, 10H), 7.27 (dt, *J*=36.3, 7.0 Hz, 7H), 7.27-7.06 (m, 3H), 7.12 (t, *J*=8.3 Hz, 3H), 6.49 (s, 1H), 6.34-6.04 (m, 3H), 5.55-5.32 (m, 2H), 5.31-4.86 (m, 4H), 4.59-4.32 (m, 1H), 4.47-3.99 (m, 3H), 3.82 (t, *J*=14.7 Hz, 2H), 3.87-3.43 (m, 4H), 3.29 (dq, *J*=15.9, 11.6, 9.5 Hz, 2H), 2.47 (d, *J*=6.5 Hz, 1H), 1.89 (ddq, *J*=28.6, 14.1, 8.1, 7.1 Hz, 4H), 1.76-1.60 (m, 1H), 1.56 (d, *J*=9.3 Hz, 1H), 1.47 (t, *J*=7.3 Hz, 2H), 1.36 (d, *J*=11.5 Hz, 1H), 1.28 (s, 1H), 0.89 (t, *J*=7.3 Hz, 3H).

DCM (50 mL) was added to the above reaction flask (**L1-D1-1**), and the mixture was dissolved with stirring. 1,8-diazabicyclo[5.4.0]undec-7-ene (DBU) (582.8 mg) was then added dropwise, and the reaction solution was stirred at room temperature for 1 h. Methyl tert-butyl ether (400 mL) was then added, and the resulting mixture was filtered. The collected solid was redissolved in DCM (40 mL), and the dissolved reaction solution was dropwise added to methyl tert-butyl ether (400 mL). The resulting mixture was filtered and dried to obtain **L1-D1-2** (7 g, yield 87.5%); LCMS: [M+1]⁺1076.38 (calculated: 1075.43); ¹H NMR (600 MHz, DMSO-*d*₆) δ 10.32 (s, 1H), 8.08-7.44 (m, 3H), 7.57-7.24 (m, 8H), 7.39-6.93 (m, 13H), 6.35-5.97 (m, 3H), 5.53-4.81 (m, 6H), 4.53-4.19 (m, 1H), 3.79 (dt,*J*=21.3, 13.7 Hz, 2H), 3.67-2.92 (m, 10H), 2.66-2.58 (m, 1H), 2.50-2.44 (m, 1H), 2.03-1.65 (m, 6H), 1.75-1.38 (m, 8H), 1.29 (dd,*J*=31.5, 16.8 Hz, 2H), 1.10 (s, 1H), 0.88 (t,*J*=7.3 Hz, 3H).

**L1-D1-2** (7 g, 6.5 mmol) and DCM (70 mL) were successively added to a 250 mL single-neck flask and dissolved with stirring. Fmoc-NH-PEG2-CH2CH2CO₂H (2.6 g, 6.5 mmol), HATU (3.7 g, 9.7 mmol) and DIPEA (1.3 g, 10 mmol) were then added. The mixture was stirred at room temperature for 1 h. The reaction was quenched with 100 mL of water, and phase separation was performed. The organic phase was washed once with a saturated sodium chloride aqueous solution (100 mL), concentrated and purified by silica gel column chromatography to obtain **L1-D1-3** (5.2 g, yield 54.7%); LCMS: [M+1]⁺1457.93 (calculated: 1456.59); ¹H NMR (600 MHz, DMSO-*d*₆) δ 9.99 (s, 2H), 8.24-7.94 (m, 2H), 7.94-7.72 (m, 2H), 7.80-7.55 (m, 2H), 7.69-7.44 (m, 2H), 7.55-7.31 (m, 5H), 7.44-7.14 (m, 14H), 7.14 (t,*J*=8.2 Hz, 1H), 6.49 (d,*J*=3.2 Hz, 1H), 6.36-6.19 (m, 2H), 5.55-5.39 (m, 3H), 5.25 (d,*J*=44.7 Hz, 2H), 5.09 (s, 1H), 5.04-4.88 (m, 1H), 4.63 (s, 1H), 4.36 (s, 1H), 4.48-3.93 (m, 4H), 3.92-3.73 (m, 2H), 3.81-3.60 (m, 2H), 3.60 (ddd,*J*=45.2, 15.2, 7.9 Hz, 6H), 3.41 (dd,*J*=49.8, 5.7 Hz, 5H), 3.11 (p,*J*=5.8 Hz, 2H), 2.72 (s, 1H), 2.37 (td,*J*=6.6, 3.3 Hz, 2H), 1.87 (dtq,*J*=21.5, 14.5, 7.1 Hz, 3H), 1.69 (s, 2H), 1.50 (d,*J*=40.9 Hz, 2H), 1.38-1.15 (m, 6H), 1.05 (d,*J*=6.4 Hz, 2H), 1.00 (d,*J*=6.5 Hz, 9H), 0.87 (t,*J*=7.4 Hz, 3H).

**L1-D1-3** (5.1 g, 3.5 mmol) and DCM (30 mL) were successively added to a 100 mL single-neck flask and dissolved with stirring. DBU (266.3 mg) was added dropwise, and the mixture was stirred at room temperature for 30 min. Methyl tert-butyl ether (300 mL) was added, and the resulting mixture was filtered. The filter cake was washed with methyl tert-butyl ether and dried to obtain **L1-D1-4 (**3.2 g, yield 74%); LCMS: [M+1]⁺1235.77 (calculated: 1234.52).

**L1-D1-4** (3.2 g, 2.6 mmol) and DCM (19 mL) were successively added to a 100 mL single-neck flask and dissolved with stirring. Bromoacetic anhydride (670 mg, 2.6 mmol) was subsequently added. The resulting mixture was stirred at room temperature for 1 h. The reaction solution was concentrated and purified by silica gel column chromatography to obtain **L1-D1-5** (2.8 g, yield 80%, HPLC 97.3%); LCMS: [M+1]⁺1355.64 (calculated: 1354.44); ¹H NMR (600 MHz, DMSO-*d*₆) δ 10.00 (s, 1H), 9.51 (s, 1H), 8.33 (d,*J*=5.6 Hz, 1H), 8.21-8.00 (m, 2H), 7.71-7.42 (m, 3H), 7.56-7.04 (m, 21H), 6.63-6.41 (m, 1H), 6.32-6.14 (m, 3H), 5.43 (s, 2H), 5.27 (d,*J*=42.2 Hz, 2H), 5.10 (s, 1H), 5.04-4.85 (m, 2H), 4.54 (d,*J*=5.1 Hz, 1H), 4.46-4.15 (m, 2H), 3.93-3.13 (m, 23H), 2.81-2.56 (m, 1H), 2.46-2.28 (m, 2H), 2.09-1.73 (m, 4H), 1.81-1.45 (m, 6H), 1.48 (d,*J*=9.5 Hz, 2H), 1.40-1.18 (m, 1H), 1.11 (s, 14H), 0.88 (t,*J*=7.3 Hz, 3H).

**L1-D1-5** (3.1 g, 2.3 mmol) and DCM (60 mL) were successively added to a 100 mL single-neck flask and dissolved with stirring. Trifluoroacetic acid (TFA) was added dropwise (the concentration of the reaction solution was 5%). The mixture was stirred at room temperature for 1 h. Methyl tert-butyl ether (600 mL) was added. The resulting mixture was filtered, washed and then dispersed in DCM (10 mL) with stirring, followed by the addition of methyl tert-butyl ether (100 mL). The mixture was stirred for 20 min, filtered, washed and then dried under vacuum to obtain white solid product **L1-D1** (1.0 g, yield 40%, HPLC 97%; LCMS: [M+1]⁺1113.51 (calculated: 1113.34); ¹H NMR (500 MHz, DMSO-*d*₆)δ10.03 (d,*J*=16.7 Hz, 1H), 8.34 (q,J=5.1 Hz, 1H), 8.23-8.10 (m, 2H), 7.86-7.60 (m, 3H), 7.72-7.40 (m, 3H), 7.33 (d,J=8.2 Hz, 1H), 7.38-6.98 (m, 2H), 6.49 (s, 1H), 6.27 (dd,*J*=13.7, 3.9 Hz, 2H), 5.43 (d,*J*=4.2 Hz, 2H), 5.33-5.18 (m, 2H), 5.10 (s, 1H), 5.03-4.91 (m, 1H), 4.51-4.20 (m, 1H), 4.36-3.88 (m, 5H), 3.93-3.11 (m, 16H), 2.78 (p,J=7.3, 6.7 Hz, 2H), 2.40 (q,*J*=5.6 Hz, 2H), 2.06-1.63 (m, 3H), 1.58 (ddt,J=45.5, 14.8, 6.1 Hz, 3H), 1.53-1.20 (m, 2H), 0.88 (t,*J*=7.2 Hz, 3H); ¹³C NMR (151 MHz, DMSO-*d*₆) δ 173.03, 171.26, 171.01, 169.51, 166.57, 158.67, 158.45, 157.32, 156.03, 155.85, 151.37, 150.58, 149.91, 149.56, 147.57, 146.75, 139.23, 139.11, 133.26, 127.13, 125.08, 119.14, 115.33, 104.56, 99.64, 96.43, 72.88, 69.92, 69.17, 67.15, 65.73, 61.82, 53.56, 50.11, 49.50, 48.21, 42.54, 39.15, 38.32, 36.31, 31.92, 30.68, 29.92, 29.04, 28.45, 27.17, 22.81, 8.25.

### 1.2 L1-D1-Ab_{nectin-4} antibody-drug conjugate (ADC1)

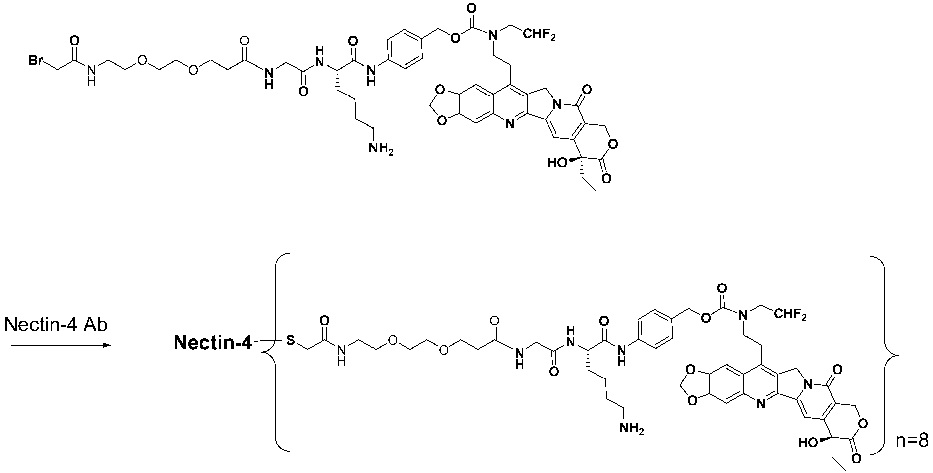

The Nectin4 antibody (10.0 mg/mL, 10 mg, 0.066 mmol) was taken, and the pH value was adjusted to 7.2 with a 1 M sodium hydrogen phosphate solution. Subsequently, a solution of 0.1 M disodium ethylenediaminetetraacetate (25 µL) and a freshly prepared TCEP·HCl solution (10 mM, 0.04 mL) were added sequentially. The reaction mixture was incubated on a rotating disk at 25°C for 3 h.

Compound **L1-D1** (0.89 mg, 0.80 mmol) was dissolved in DMA (0.09 mL). The resulting solution was added to the above solution system and mixed thoroughly. The mixture incubated on a rotating disk at room temperature for 16 h. Upon completion of the reaction, small molecules were removed by using NAP-5 gel column (Cytiva), and the buffer was exchanged into a 20 mM PB solution (pH = 6.3) to obtain the antibody-drug conjugate **ADC1** (3.1 mg/mL, 2 mL).

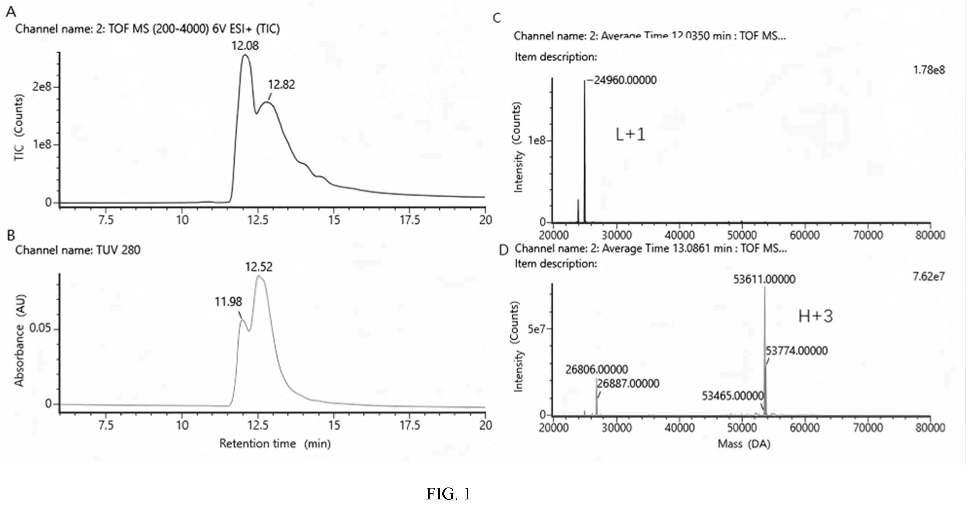

RP-MS (RP-HPLC-MS) calculated: average: n=7.7; MS results showed that the light chain (L) of the antibody was linked to one **L1-D1** (linker-payload), and the heavy chain (H) was linked to three **L1-D1** (linker-payload) (FIG. 1).

### Example 2: Ab_{nectin-4}-(S-7-(N-(acetamide-PEG₂-propionyl-Gly-Lys-PABC)-N-((R)-3-tetrahydrofuryl))aminoethyl camptothecin)₈ (ADC2)

### 2.1 7-(N-(bromoacetamide-PEG₂-propionyl-Gly-Lys-PABC)-N-((R)-3-tetrahydrofuranyl))aminoethyl camptothecin (L1-D2)

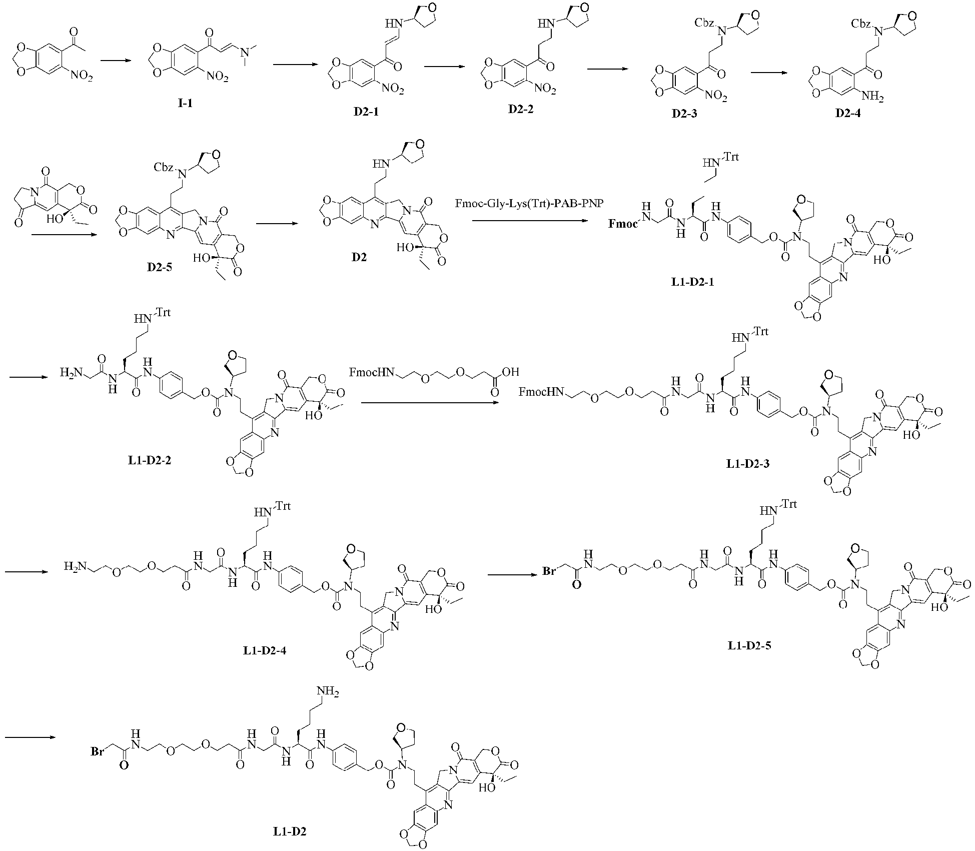

Intermediate **I-1** (18 g, 68.12 mmol) was added into isopropyl alcohol (IPA) (180 mL), and the reaction solution was stirred. DIEA (17.61 g, 136.24 mmol) and (R)-3-aminotetrahydrofuran hydrochloride (33.67 g, 272.48 mmol) were then added. The mixture was heated under reflux for 12 h. The reaction solution was concentrated to a residual volume of approximately 20 mL of IPA, followed by adding 200 mL of water. The resulting reaction solution was stirred at room temperature for 2 h and then filtered to obtain a yellow solid. The solid was dried at 40°C to obtain product **D2-1** (16.5 g, yield 79%, HPLC 98%); LCMS: [M+1]⁺307.32 (calculated: 306.09); ¹H NMR (500 MHz, DMSO-*d*₆) δ 7.58 (s, 1H), 7.34-7.19 (m, 1H), 7.07 (s, 1H), 6.25 (s, 2H), 5.33 (d,*J*=11.3 Hz, 1H), 4.02 (s, 1H), 3.83-3.53 (m, 5H), 2.23-2.05 (m, 1H), 1.85-1.65 (m, 1H).

Compound **D2-1** (16.5 g, 53.87 mmol) was added to DCM (310 mL), and acetic acid (64.70 g, 1.08 mol) was subsequently added. The mixture was dissolved with stirring. The internal temperature of the reaction solution was maintained at 15°C, and sodium triacetoxyborohydride (28.54 g, 134.68 mmol) was added in four batches of approximately 7 g at an interval of 5 min. The reaction was carried out at a constant internal temperature of 15°C for 2 h. The reaction solution was poured into an ice-cold sodium carbonate aqueous solution (sodium carbonate: 130 g, and ice water: 1 L) while the internal temperature was controlled at 15°C during stirring. The phase separation was performed. The organic phase was washed with water. The collected organic phase was dried over anhydrous sodium sulfate to obtain a solution of compound **D2-2** in DCM. DIEA (13.93 g, 107.76 mmol) was added to the above solution, followed by the dropwise addition of Cbz-Cl (10.11 g, 59.27 mmol) over 20 min. The mixture reacted for 12 h under ice-cooling conditions. The reaction product was washed twice with a 10% citric acid aqueous solution (200 mL *2), and then sequentially washed with a aqueous sodium bicarbonate aqueous solution (200 mL) and saturated brine (200 mL). The organic phase was dried over anhydrous sodium sulfate and concentrated to obtain compound **D2-3** (20.5 g, yield 86%); LCMS: [M+1]⁺443.42 (calculated: 442.43); ¹H NMR (500 MHz, DMSO-d₆) δ 7.65 (s, 1H), 7.39-7.27 (m, 6H), 6.28 (s, 2H), 5.10 (s, 2H), 4.65-4.50 (m, 1H), 4.00-3.80 (m, 1H), 3.77-3.57 (m, 5H), 3.16-3.05 (m, 2H), 2.24-2.10 (m, 1H), 2.00-1.88 (m, 1H).

Compound **D2-3** (20.5 g, 46.3 mmol) was added to a 1 L single-neck flask, followed by the addition of DMF (50 mL). The reaction system was cooled to 0°C in an ice bath under nitrogen protection. A freshly prepared reducing solution consisting of water (250 mL)/sodium dithionite (40.33 g, 231.68 mmol)/sodium carbonate (19.64 g, 185.34 mmol) was slowly added dropwise. Upon completion of the addition, the reaction system was slowly heated to 40°C and then further stirred for 2 h. The reaction solution was filtered, and the filtrate was concentrated. Ethyl acetate (500 mL) and water (500 mL) were added for extraction and phase separation. The organic phase was dried over anhydrous sodium sulfate, concentrated, and purified by silica gel column chromatography to obtained yellow oil **D2-4** (17.9 g, yield 93.7%, HPLC 98%); LCMS: [M+1]⁺413.42 (calculated: 412.44); ¹H NMR (500 MHz, DMSO-d₆) δ 7.45-7.25 (m, 7H), 5.94 (s, 2H), 5.08 (s, 2H), 4.60-4.45 (m, 1H), 3.90-3.80 (m, 1H),3.77-3.41(m, 5H), 3.05 (t,*J*=7.6 Hz, 2H), 2.19-2.10 (m, 1H), 1.87 (d,*J*=7.9 Hz, 1H).

Compound **D2-4** (17.9 g, 43.4 mmol), (S)-4-ethyl-4-hydroxy-7,8-dihydro-1H-pyrano [3,4-f] indolizine-3,6,10 (4H)-trione (12.57 g, 47.74 mmol) and PPTS (218.13 g, 868.00 mmol) were successively added to a 500 mL single-neck flask. Under argon protection, the mixture was stirred and heated at 110°C for 12 h. The reaction solution was poured into methanol (1000 mL) and stirred for 2 h, and then filtered to obtain a dark brown solid. The obtained solid was dispersed in methanol (300 mL) and stirred for 2 h, followed by filtration to obtain a light brown solid. The light brown solid was dried at 40°C to obtain compound **D2-5** (10.0 g, yield 36%, HPLC 95%): LCMS: [M+1]⁺640.72 (calculated: 639.66); ¹H NMR (600 MHz, DMSO-d₆) δ 7.82-7.14 (m, 8H), 6.49 (s, 1H), 6.29 (s, 2H), 5.53-5.03 (m, 5H), 4.60 (s, 1H), 4.08-3.23 (m, 9H), 2.25-1.73 (m, 4H), 0.88 (s, 3H).

Compound **D2-5** (10 g, 15.63 mmol) was added to a 10 L single-neck flask, followed by the addition of dichloromethane (2.5 L) and methanol (2.5 L). 10% Pd/C (15 g) was added to the reaction solution. The reaction was carried out at room temperature under a hydrogen balloon atmosphere for 20 h. The Pd/C catalyst was removed by filtration. The reaction solution was evaporated to dryness. The resulting residue was stirred in a mixed solution of dichloromethane and methanol (30 mL, V/V = 1:1) for 24 h and then filtered. The wet product was dried at 40°C for 3 h to obtain product **D2** (6.0 g, yield 76%); LCMS: [M+1]⁺506.52 (calculated: 505.53); ¹H NMR (600 MHz, DMSO-d₆) δ 7.52 (s, 1H), 7.40 (s, 1H), 7.16 (s, 1H), 6.52 (s, 1H), 6.26 (d,*J*=7.3 Hz, 2H), 5.48-5.35 (m, 2H), 5.11-4.95 (m, 2H), 3.95-3.62 (m, 5H), 3.36-3.26 (m, 2H), 3.14-3.03 (m, 2H), 2.20-2.04 (m, 2H), 1.96-1.80 (m, 2H), 0.89 (t,*J*=7.3 Hz, 3H).

**D2** (7.6 g, 15.03 mmol), NMP (140 mL), DIPEA (5.83 g, 45.1 mmol), Fmoc-Gly-Lys(Trt)-PAB-PNP (12.69 g, 13.53 mmol) and HOBT (2.23 g, 16.54 mmol) were successively added to a 250 mL single-neck flask. The reaction solution was stirred at room temperature for 2 h. Dichloromethane (300 mL) was added. The organic phase was washed with a 2% citric acid aqueous solution, a saturated sodium bicarbonate aqueous solution (100 mL) and saturated brine in sequence, dried over anhydrous sodium sulfate, and concentrated to obtain **L1-D2-1;** LCMS: [M+1]⁺1304.93 (calculated: 1303.53);¹H NMR (600 MHz, DMSO-*d*₆) δ 10.06 (s, 1H), 8.10 (d,*J*=7.9 Hz, 1H), 7.92-7.73 (m, 2H), 7.71-7.51 (m, 4H), 7.44-7.18 (m, 18H), 7.12 (t,*J*=7.1 Hz, 3H), 6.49 (s, 1H), 6.31-6.20 (m, 2H), 5.42 (d,*J*=3.5 Hz, 2H), 5.23 (s, 2H), 5.14 (s, 2H), 4.58 (s, 1H), 4.41 (s, 1H), 4.27-4.17 (m, 2H), 3.94 (dd,*J*=40.6, 15.3 Hz, 1H), 3.73-3.42 (m, 5H), 3.30 (t,*J*=7.1 Hz, 2H), 3.07 (d,*J*=0.8 Hz, 4H), 2.69 (d,*J*=1.0 Hz, 2H), 2.47 (s, 1H), 2.21-2.08 (m, 2H), 1.94-1.79 (m, 4H), 1.72-1.39 (m, 3H), 1.39-1.22 (m, 2H), 1.10 (d,*J*=0.8 Hz, 11H), 0.88 (t,*J*=7.3 Hz, 3H).

**L1-D2-1** (the above product) and DCM (100 mL) were added to a 250 mL single-neck flask and dissolved with stirring. DBU (3.61 g, 23.7 mmol) was added dropwise, and the mixture was stirred at room temperature for 1 h. The resulting reaction solution was poured into methyl tert-butyl ether (1 L) to precipitate a solid. The collected solid was redissolved in DCM (80 mL), and the resulting solution was dropwise added to 1 L of methyl tert-butyl ether. The mixture was filtered, and the solid was dried to obtain **L1-D2-2** (26 g); LCMS: [M+1]⁺1082.68 (calculated: 1081.47);¹H NMR (600 MHz, DMSO-*d*₆) δ 10.30 (d,*J*=65.6 Hz, 1H), 7.82 (d,*J*=8.3 Hz, 1H), 7.66-7.59 (m, 1H), 7.50-7.45 (m, 1H), 7.41-7.34 (m, 9H), 7.31-7.20 (m, 8H), 7.14 (t,*J*=7.1 Hz, 4H), 6.70-6.63 (m, 1H), 6.37-6.05 (m, 2H), 5.44-5.38 (m, 2H), 5.22 (s, 3H), 5.13 (s, 2H), 4.58 (s, 1H), 4.43 (d,*J*=9.2 Hz, 1H), 4.27 (s, 1H), 4.08-3.77 (m, 1H), 3.76-3.52 (m, 2H), 3.50-3.31 (m, 5H), 3.19 (t,*J*=5.8 Hz, 2H), 3.10 (s, 1H), 2.89 (s, 1H), 2.73 (d,*J*=0.7 Hz, 1H), 2.63-2.57 (m, 1H), 2.19-2.05 (m, 1H), 1.99-1.70 (m, 6H), 1.58 (ddd,*J*=34.2, 9.8, 5.4 Hz, 7H), 1.46 (d,*J*=7.7 Hz, 2H), 0.92-0.83 (m, 3H).

**L1-D2-2** (26 g of crude product) and DCM (400 mL) were successively added to a 1 L single-neck flask and dissolved with stirring. Fmoc-NH-PEG2-CH2CH2COOH (6.2 g, 15.6 mmol), HATU (13.7 g, 36.0 mmol) and DIPEA (4.6 g, 36.0 mmol) were then added. Upon completion of the addition, the mixture was stirred at room temperature for 1 h. The reaction was quenched with 80 mL of water, and the mixture was extracted with DCM. The organic phase was washed with saturated brine and dried over anhydrous sodium sulfate. The crude product was purified by silica gel column chromatography to obtain **L1-D2-3** (11.0 g, total yield for three steps: 55.5%, HPLC purity: 96%); LCMS: [M+1]⁺1463.78 (calculated: 1462.62);¹H NMR (600 MHz, DMSO-*d*₆) δ 10.01 (s, 1H), 8.17-8.04 (m, 2H), 7.85 (d,*J*=7.5 Hz, 2H), 7.65 (dd,*J*=20.1, 7.9 Hz, 4H), 7.53-7.18 (m, 20H), 7.14 (q,*J*=7.5, 5.7 Hz, 3H), 6.49 (s, 1H), 6.26 (d,*J*=16.8 Hz, 2H), 5.42 (d,*J*=3.2 Hz, 2H), 5.23 (s, 2H), 5.13 (s, 2H), 4.58 (s, 1H), 4.38 (q,*J*=7.6 Hz, 1H), 4.26 (d,*J*=7.1 Hz, 2H), 4.17 (t,*J*=6.9 Hz, 1H), 3.97 (s, 0H), 3.90 (d,*J*=17.7 Hz, 0H), 3.71 (dd,*J*=31.9, 4.9 Hz, 3H), 3.60 (dt,*J*=21.8, 6.7 Hz, 4H), 3.45 (s, 4H), 3.37 (t,*J*=6.0 Hz, 2H), 3.27 (s, 2H), 3.11 (p,*J*=6.7, 6.0 Hz, 2H), 2.36 (t,*J*=6.5 Hz, 2H), 2.13 (dtd,*J*=13.3, 8.5, 5.4 Hz, 1H), 1.87 (ddt,*J*=36.3, 21.3, 7.1 Hz, 4H), 1.66 (s, 1H), 1.53 (d,*J*=10.0 Hz, 1H), 1.46 (q,*J*=7.2 Hz, 2H), 1.39-1.20 (m, 3H), 0.87 (t,*J*=7.3 Hz, 3H).

**L1-D2-3** (5 g, 3.42 mmol) and DCM (40 mL) were successively added to a 100 mL single-neck flask and dissolved with stirring. DBU (364 mg, 2.39 mmol) was added dropwise. Upon completion of the addition, the mixture was stirred at room temperature for 30 min. The reaction solution was poured into methyl tert-butyl ether (500 mL), and the solid was precipitated by filtration. The obtained solid was redissolved in DCM (50 mL), and the resulting solution was dropwise added to methyl tert-butyl ether (600 mL). The mixture was filtered, and the filter cake was washed with methyl tert-butyl ether and dried to obtain **L1-D2-4** (2.9 g, yield 69%); LCMS: [M+1]⁺1241.87 (calculated: 1240.55);¹H NMR (600 MHz, DMSO-*d*₆) δ 10.03 (s, 1H), 8.14 (t,*J*=5.7 Hz, 1H), 8.09 (d,*J*=7.8 Hz, 1H), 7.63 (d,*J*=8.3 Hz, 2H), 7.48 (s, 1H), 7.41 (s, 1H), 7.37 (d,*J*=7.9 Hz, 7H), 7.28-7.19 (m, 7H), 7.14 (t,*J*=7.3 Hz, 3H), 6.49 (s, 1H), 6.27 (d,*J*=11.9 Hz, 2H), 5.48-5.37 (m, 2H), 5.24 (s, 2H), 5.14 (s, 2H), 4.58 (t,*J*=7.5 Hz, 1H), 4.37 (q,*J*=7.3 Hz, 1H), 3.89 (s, 1H), 3.71 (dd,*J*=24.3, 7.2 Hz, 3H), 3.60 (dt,*J*=22.9, 7.1 Hz, 4H), 3.46 (s, 4H), 3.36 (t,*J*=5.7 Hz, 2H), 2.67 (t,*J*=5.7 Hz, 2H), 2.37 (t,*J*=6.5 Hz, 2H), 2.13 (dtd,*J*=13.2, 8.6, 5.1 Hz, 1H), 1.88 (dtd,*J*=43.1, 13.7, 13.2, 6.6 Hz, 4H), 1.66 (s, 1H), 1.50 (dp,*J*=37.5, 7.7 Hz, 4H), 1.39-1.14 (m, 5H), 0.88 (t,*J*=7.3 Hz, 3H).

**L1-D2-4** (2.9 g, 2.3 mmol) and DCM (25 mL) were successively added to a 100 mL single-neck flask and dissolved with stirring. Bromoacetic anhydride (667.8 mg, 2.57 mmol) was then added, and the mixture was stirred at room temperature for 30 min. The reaction solution was concentrated and purified by silica gel column chromatography to obtain **L1-D2-5** (2.0 g, yield 64.5%, HPLC 97.6%); LCMS: [M+1]⁺1361.93 (calculated: 1360.48);¹H NMR (600 MHz, DMSO-*d*₆) δ 10.01 (s, 1H), 8.32 (t,*J*=5.6 Hz, 1H), 8.11 (dd,*J*=24.1, 6.7 Hz, 2H), 7.62 (d,*J*=8.1 Hz, 2H), 7.55-7.20 (m, 17H), 7.14 (s, 2H), 6.49 (s, 1H), 6.26 (s, 2H), 5.42 (d,*J*=2.8 Hz, 2H), 5.24 (s, 2H), 5.14 (s, 2H), 4.58 (p,*J*=6.4, 5.8 Hz, 1H), 4.40-4.34 (m, 1H), 3.96-3.86 (m, 0H), 3.84 (s, 2H), 3.71 (dd,*J*=22.2, 5.6 Hz, 3H), 3.58 (t,*J*=6.5 Hz, 3H), 3.47 (s, 5H), 3.43-3.31 (m, 5H), 3.21 (q,*J*=5.8 Hz, 2H), 2.37 (t,*J*=6.5 Hz, 2H), 2.14 (dtd,*J*=13.4, 8.7, 5.3 Hz, 1H), 1.87 (dtd,*J*=28.6, 15.1, 14.2, 7.9 Hz, 4H), 1.66 (td,*J*=12.2, 6.1 Hz, 2H), 1.51 (d,*J*=42.3 Hz, 3H), 1.35-1.24 (m, 5H), 0.91-0.81 (m, 3H).

**L1-D2-5** (1.3 g, 0.95 mmol) and DCM (10 mL) were successively added to a 100 mL single-neck flask and dissolved with stirring. TFA (0.78 mL) was added dropwise, and the mixture was stirred at room temperature for 2 h. The reaction solution was poured into methyl tert-butyl ether (130 mL), and the solid was precipitated by filtration. The obtained solid was dispersed in DCM (10 mL) with stirring, and then poured into methyl tert-butyl ether (100 mL). The mixture was stirred for 20 min. The organic phase was filtered, washed and dried under vacuum to obtain **L1-D2** (870 mg, yield 81.7%, HPLC 97%); LCMS: [M+1]⁺1119.64 (calculated: 1118.36); ¹H NMR (500 MHz, DMSO-d₆) δ 10.05 (s, 1H), 8.34 (s, 1H), 8.26-8.09 (m, 2H), 7.73 (s, 3H), 7.65 (d,*J*=8.2 Hz, 2H), 7.47 (s, 1H), 7.40 (s, 2H), 7.22 (s, 1H), 6.49 (s, 1H), 6.27 (s, 2H), 5.43 (s, 2H), 5.30-5.05 (m, 4H), 4.65-4.55 (m, 1H), 4.45-4.35 (m, 1H), 3.89-3.57 (m, 16H), 3.44-3.18 (m, 6H), 2.85-2.70 (m, 2H), 2.40 (t,*J*=6.3 Hz, 2H), 2.20-2.10 (m, 1H), 1.96-1.70 (m, 4H), 1.68-1.25 (m, 5H), 0.89 (t,*J*=7.3 Hz, 3H).

### 2.2 L1-D2-Ab_{nectin-4} antibody-drug conjugate (ADC2)

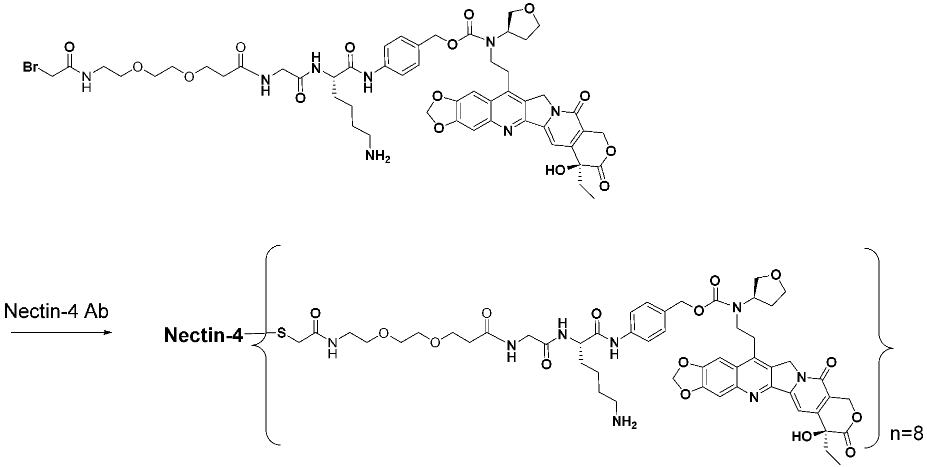

The Nectin4 antibody (10.0 mg/mL, 10 mg, 0.066 mmol) was taken, and the pH was adjusted to 7.2 with a 1 M disodium hydrogen phosphate solution. Subsequently, 0.1 M disodium ethylenediaminetetraacetate solution (25 µL) and a freshly prepared TCEP•HCl (tris(2-carboxyethyl)phosphine hydrochloride) solution (10 mM, 0.04 mL) were added sequentially. The mixture was incubated on a rotating disk at 25°C for 3 h.

Compound **L1-D2** (0.90 mg, 0.80 mmol) was dissolved in 0.09 mL of DMA. The resulting solution was added to the above solution system and mixed thoroughly. The mixture was incubated on a rotating disk at room temperature for 16 h. Upon completion of the reaction, small molecules were removed by using a NAP-5 gel column (Cytiva), and the buffer was exchanged into a 20 mM PB solution (pH = 6.3) to obtain the antibody-drug conjugate **ADC2.** (3.1 mg/mL, 2 mL).

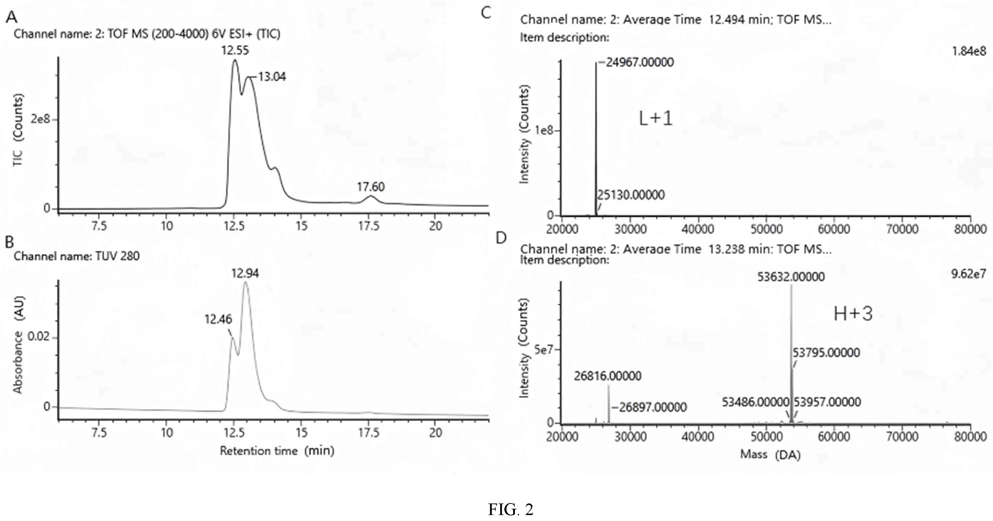

RP-MS calculated: average: n=7.9; MS results showed that the light chain (L) of the antibody was linked to one **L1-D2** (linker-payload), and the heavy chain (H) of the antibody was linked to three **L1-D2** (linker-payload) (FIG. 2).

### 2.3 7-(N-(bromoacetamide-PEG₈-propionyl-Gly-Lys-PABC)-N-((R)-3-tetrahydrofuranyl))aminoethyl camptothecin (L2-D2)

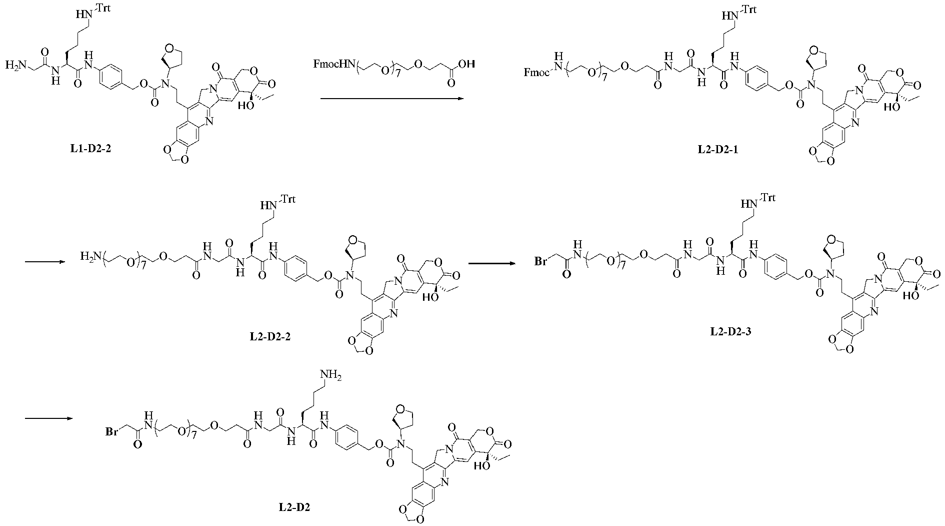

**L1-D2-2** (1.0 g, 924.02 µmol), DMF (10 mL), Fmoc-PEG8-OSu (703.03 mg, 924.02 µmol) and DIPEA (116.11 mg, 924.02 µmol, 156 µL) were successively added to a 100 mL single-neck flask at room temperature. The mixture was stirred at room temperature for 1 h, then concentrated and purified by silica gel column chromatography to obtain yellow solid **L2-D2-1** (1.26 g, yield 78.25%); LCMS: [M+1]⁺1728.85 (calculated: 1727.97).

**L2-D2-1** (1.26 g, 729.59 µmol), DMF (10 mL) and piperidine (861.9 mg, 10.14 mmol, 1.0 mL) were successively added to a 10 mL single-neck round-bottom flask at room temperature. The mixture was stirred at room temperature for 20 min, concentrated, and purified by silica gel column chromatography to obtain light yellow solid **L2-D2-2** (960 mg, yield 87.27%); LCMS: [M+1]⁺1506.38 (calculated: 1505.73).

**L2-D2-2** (960 mg, 637.57 µmol), DMF (10 mL) and bromoacetic anhydride (165.69 mg, 637.57 µmol) were successively added to a 10 mL single-neck flask at room temperature. The mixture was stirred at room temperature for 30 min, then concentrated and purified by silica gel column chromatography to obtain light yellow solid **L2-D2-3** (440 mg, yield 42.31%); LCMS: [M+1]⁺1627.51 (calculated: 1626.66).

**L2-D2-3** (440 mg, 270.61 µmol), DCM (5 mL) and TFA (744.52 mg, 6.53 mmol, 500 µL) were successively added to a 10 mL single-neck round-bottom flask at room temperature. The mixture was stirred at room temperature for 2 h. Diethyl ether (10 mL) was added dropwise to the reaction solution. The mixture was centrifuged at 10000 rpm for 5 min, and the supernatant was discarded. The solid was evaporated to dryness to remove the residual solvent to obtain yellow solid powder L2-D2 (350 mg, yield 93.58%, HPLC 97%); LCMS: [M+1]⁺1385.22 (calculated: 1384.34); ¹H NMR (600 MHz, DMSO-*d*₆) δ 10.04 (s, 1H), 8.35 (t,*J*=5.7 Hz, 1H), 8.19 (t,*J*=5.7 Hz, 1H), 8.15 (d,*J*=8.0 Hz, 1H), 7.72 (d,*J*=5.8 Hz, 3H), 7.65 (d,*J*=8.1 Hz, 2H), 7.49 (s, 1H), 7.39 (s, 2H), 7.23 (s, 1H), 6.28 (s, 2H), 5.47-5.38 (m, 2H), 5.22 (s, 2H), 5.14 (s, 2H), 4.58 (p,*J*=5.8, 5.4 Hz, 1H), 4.41 (q,*J*=7.7, 7.2 Hz, 1H), 3.93 (s, 1H), 3.89 (s, 1H), 3.86 (s, 2H), 3.81-3.68 (m, 4H), 3.67-3.56 (m, 5H), 3.53-3.48 (m, 19H), 3.46-3.35 (m, 3H), 3.25 (dq,*J*=17.2, 5.4 Hz, 3H), 2.78 (q,*J*=6.9, 6.3 Hz, 2H), 2.40 (dt,*J*=8.2, 4.1 Hz, 2H), 2.14 (dtt,*J*=13.2, 8.7, 5.5 Hz, 1H), 1.87 (dh,*J*=21.2, 7.3 Hz, 2H), 1.76 (ddt,*J*=16.4, 10.4, 5.3 Hz, 1H), 1.62 (qd,*J*=13.3, 11.3, 6.8 Hz, 1H), 1.55 (dp,*J*=14.0, 6.6 Hz, 2H), 1.39 (td,*J*=8.9, 5.4 Hz, 1H), 1.36-1.29 (m, 1H), 1.09 (t,*J*=7.0 Hz, 2H), 0.89 (t,*J*=7.3 Hz, 3H).

### Example 3: Ab_{nectin-4}-(S-7-(N-(acetamide-PEG₂-propionyl-Gly-Lys-PAB(3-bis-PEG₄-carbonyl)C)-N-((R)-3-tetrahydrofuryl))aminoethyl camptothecin)s (ADC3)

### 3.1 7-(N-(bromoacetamide-PEG₂-propionyl-Gly-Lys-PAB(3-bis-PEG₄-carbonyl)C)-N-((R)-3-tetrahydrofuranyl))aminoethyl camptothecin (L3-D2)

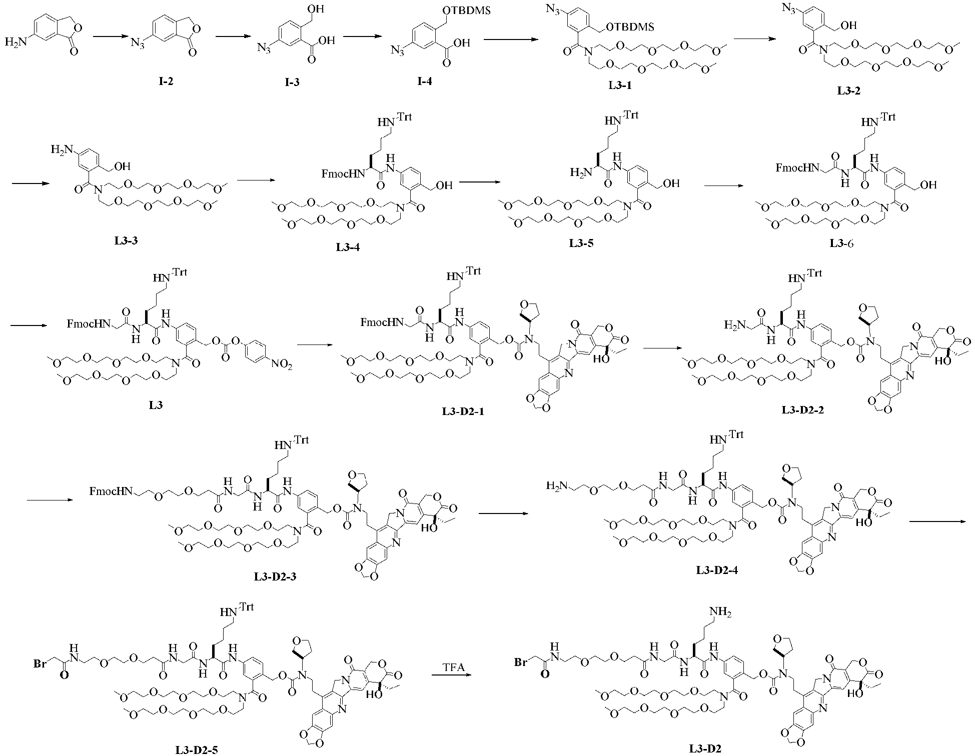

6-amino-3H-isobenzofuran-1-one (4.0 g, 26.82 mmol), acetonitrile (50 mL), imidazole-1-sulfonyl azide hydrochloride (6.75 g, 32.18 mmol), CuSO₄ (5.14 g, 32.18 mmol) and potassium carbonate (37.07 mg, 268.19 µmol) were added to a reaction flask. The reaction solution was stirred at room temperature for 16 h. The insoluble solids were removed by filtration, and the filtrate was concentrated. The residue was extracted with dichloromethane. The organic phase was washed with saturated brine, dried over anhydrous sodium sulfate and concentrated to obtain intermediate **I-2** (2.0 g, yield 38%), which was used in the next step without purification.

**I-2** (6.9 g, 39.40 mmol), THF (50 mL), water (50 mL) and NaOH (2.36 g, 59.09 mmol) were added to a reaction flask. The reaction solution was stirred at 70°C for 2 h and concentrated under reduced pressure. The residual solution was acidified with 1 N HCl, and the precipitated white solid was collected by filtration and dried to obtain 5-azido-2-hydroxymethylbenzoic acid **I-3** (7.3 g, yield 91%).

**I-3** (7.0 g, 36.24 mmol), DMF (150 mL), imidazole (12.34 g, 181.20 mmol) and TBDMS-Cl (13.66 g, 90.60 mmol) were added to a reaction flask at 0°C. The mixture was stirred at room temperature overnight and then poured into water. The precipitated solid was collected by filtration, washed with water, and dried to obtain gray-white solid product 5-azido-2-(TBDMSO)methylbenzoic acid **I-4** (10 g, yield 88%). ¹H NMR (600 MHz, CDCl₃) δ 7.68-7.51 (m, 2H), 7.10 (dd,*J* = 8.4, 2.6 Hz, 1H), 4.92 (d,*J* = 0.9 Hz, 2H), 0.82 (s, 9H), 0.00 (s, 6H).

**N,N-bis(methoxyethoxyethoxyethoxyethyl)amine or (Me-PEG₄)₂NH**: Methoxyethoxyethoxyethoxyethanol (40 g, 191.2 mmol), triethylamine (58 g, 567.8 mmol), p-toluenesulfonyl chloride (43.7 g, 228.2 mmol) and DCM (400 mL) were added to a reaction flask. The reaction solution was stirred at room temperature for 3 h and concentrated under reduced pressure. The crude product was purified by silica gel column chromatography to obtain oily product methoxyethoxyethoxyethoxyethanol tosylate (61.5 g, yield 88%); LCMS: [M+H]⁺363.50 (calculated: 362.44). N-benzyl-N,N-bis(methoxyethoxyethoxyethoxyethyl)amine tosylate (58.5 g, 161 mmol), benzylamine (8.7 g, 80.5 mmol) and K₂CO₃ (33.4 g, 241.4 mmol) were added to a reaction flask containing acetonitrile (500 mL). The reaction mixture was heated to 70°C with stirring for 16 h. The reaction product was filtered, and the filtrate was concentrated. The crude product was purified by silica gel column chromatography to obtain N-benzyl-N,N-bis(methoxyethoxyethoxyethoxyethyl)amine (23.1 g, yield 58.7%); LCMS: [M+H]⁺488.54 (calculated: 487.63). N-benzyl-N,N-bis(methoxyethoxyethoxyethoxyethyl)amine (10 g, 20.5 mmol), methanol (100 mL) and 10% Pd-C were added into a reaction flask. The reaction solution was stirred at room temperature for 16 h under a hydrogen balloon atmosphere. The reaction product was filtered, and the filtrate was concentrated to obtain oily product N,N-bis(methoxyethoxyethoxyethoxyethyl)amine, **(Me-PEG4)₂NH** (7.9 g, yield 97%); LCMS: [M+H]⁺398.31 (calculated: 397.27).

**I-4** (10.5 g, 34.16 mmol), (Me-PEG4)₂NH (14.93 g, 37.57 mmol), DCM (250 mL), HATU (19.48 g, 51.23 mmol) and triethylamine (17.28 g, 170.78 mmol, 23.80 mL) were added to a reaction flask. The reaction mixture was stirred at room temperature for 4 h. Water was added, and the reaction solution was extracted with DCM. The organic phase was dried over anhydrous sodium sulfate, concentrated, and purified by silica gel column chromatography to obtain colorless liquid product 5-azido-2-(TBDMSO)methylbenzoyl bis(Me-PEG4)amine **L3-1** (15 g, yield 59.46%); LCMS: [M+Na]⁺709.42 (calculated: 686.39); ¹H NMR (500 MHz, CDCl₃) δ 7.44 (d,*J*=8.4 Hz, 1H), 6.96 (dd,*J*=8.3, 2.4 Hz, 1H), 6.81 (d,*J*=2.4 Hz, 1H), 4.58 (s, 2H), 3.66 (s, 3H), 3.61-3.49 (m, 18H), 3.48-3.37 (m, 8H), 3.36-3.22 (m, 8H), 0.84 (s, 9H), 0.03 (s, 6H).

**L3-1** (15 g, 21.84 mmol), DCM (250 mL) and TBAF (12.21 g, 43.67 mmol) were added to a reaction flask. The mixture was stirred at room temperature for 16 h and poured into water. The reaction solution was extracted with DCM, and the organic phase was washed with saturated brine and dried over anhydrous sodium sulfate. The crude product was purified by silica gel column chromatography to obtain colorless liquid product 5-azido-2-hydroxymethylbenzoyl bis(Me-PEG4)amine **L3-2** (11 g, yield 83.6%); LCMS: [M+H]⁺573.32 (calculated: 572.31); ¹H NMR (500 MHz, CDCl₃) δ 7.44 (d,*J*=8.2 Hz, 1H), 7.03 (dd,*J*=8.2, 2.4 Hz, 1H), 6.95 (d,*J*=2.3 Hz, 1H), 4.55 (s, 2H), 3.81-3.77 (m, 2H), 3.72-3.58 (m, 21H), 3.57-3.48 (m, 10H), 3.37 (d,*J*=6.6 Hz, 6H).

**L3-2** (11 g, 16.01 mmol), MeOH (50 mL), DIPEA (2.07 g, 16.01 mmol) and 10% Pd-C (1.5 g) were added into a reaction flask. The reaction solution was stirred under a hydrogen balloon atmosphere at atmospheric pressure for 2 h. The reaction product was filtered and concentrated to obtain colorless liquid product 5-amino-2-hydroxymethylbenzoyl bis(Me-PEG4)amine **L3-3** (9 g, yield 93.2%); LCMS: [M+Na]⁺569.20 (calculated: 546.32); ¹H NMR (500 MHz, DMSO-d₆) δ 7.13 (d,*J*=8.3 Hz, 1H), 6.58 (dd,*J*=8.2, 2.3 Hz, 1H), 6.38 (d,*J*=2.4 Hz, 1H), 5.13 (s, 2H), 4.70 (t,*J*=5.5 Hz, 1H), 4.27 (d,*J*=5.5 Hz, 2H), 3.63 (d,*J*=5.0 Hz, 2H), 3.61-3.49 (m, 19H), 3.49-3.43 (m, 8H), 3.34 (t,*J*=6.0 Hz, 3H), 3.27 (d,*J*=1.0 Hz, 6H).

Fmoc-Lys(Trt) (366 mg, 0.60 mmol), **L3-3** (500 mg, 0.92 mmol) and DCM (5 mL) were added to a 50 mL single-neck flask. The reaction mixture was cooled in an ice bath, and EEDQ (222 mg, 0.90 mmol) was added to the reaction flask. The resulting solution was further stirred in an ice bath for 12 h, and then poured into a saturated sodium bicarbonate aqueous solution. The reaction solution was extracted with DCM, and the organic phase was washed with saturated brine, dried over anhydrous sodium sulfate and concentrated to obtain crude product **L3-4**, which was used directly in the next step; LCMS: [M+1]⁺1139.61 (calculated: 1138.59).

The above **L3-4** (about 1 g, crude product) was dissolved in DCM (mL) and transferred to a single-neck flask. DBU (mg, 0.00 mmol) was added under stirring, and the mixture reacted at room temperature for 30 min. The reaction solution was evaporated to dryness directly to obtain crude product **L3-5** (about 900 mg), which was used directly in the next step; LCMS: [M+H]⁺917.91 (calculated: 916.52).

The **L3-5** (about 900 mg, crude product) was dissolved in DCM (10 mL) and transferred into a 50 mL single-neck flask. The reaction solution was cooled in an ice bath, followed by the sequential addition of PPTS (108 mg, 0.43 mmol), Fmoc-glycine (108 mg, 0.36 mmol), EDCI (173 mg, 0.90 mmol) and HOBT (120 mg, 0.89 mmol). The mixture was stirred at room temperature for 2 h. Water was added, and the reaction solution was extracted with DCM. The organic phase was washed with a saturated sodium bicarbonate aqueous solution and saturated brine, dried over anhydrous sodium sulfate, and concentrated. The crude product was purified by silica gel column chromatography to obtain yellow oil **L3-6** (300 mg, total yield over three steps 41.8%); LCMS: [M+1]⁺1196.87 (calculated: 1195.61); ¹H NMR (600 MHz, DMSO-*d*₆) δ 10.04 (s, 1H), 8.10 (d,*J* = 7.8 Hz, 1H), 7.88 (d,*J* = 7.6 Hz, 1H), 7.69 (d,*J* = 7.5 Hz, 1H), 7.54 (t,*J* = 6.1 Hz, 2H), 7.50-7.45 (m, 1H), 7.45-7.35 (m, 8H), 7.32 (q,*J* = 7.5, 7.0 Hz, 2H), 7.25 (t,*J* = 7.7 Hz, 5H), 7.14 (t,*J* = 7.4 Hz, 3H), 5.12-5.03 (m, 1H), 4.38 (d,*J* = 5.7 Hz, 3H), 4.30-4.12 (m, 3H), 3.73-3.58 (m, 5H), 3.55 (q,*J* = 2.1 Hz, 5H), 3.56-3.27 (m, 25H), 3.25-3.18 (m, 8H), 1.92 (q,*J* = 7.2 Hz, 2H), 1.72-1.62 (m, 1H), 1.60-1.51 (m, 1H), 1.47 (p,*J* = 7.6 Hz, 2H), 1.38-1.28 (m, 1H), 1.29-1.22 (m, 3H).

**L3-6** (300 mg, 0.25 mmol) and DCM (3 mL) were added to a 50 mL single-neck flask at room temperature and dissolved with stirring. TEA (75 mg, 0.75 mmol) and bis(4-nitrophenyl) carbonate (228 mg, 0.75 mmol) were added dropwise, and the mixture was stirred at room temperature for 2 h. The reaction solution was poured into water and extracted with DCM. The organic phase was washed with saturated brine, dried over anhydrous sodium sulfate, and evaporated to dryness. The crude product was purified by silica gel column chromatography to obtain **L3** (280 mg, yield 82%); LCMS: [M+1]⁺ 1361.64 (calculated: 1360.62); ¹H NMR (600 MHz, DMSO-*d*₆) δ 10.20 (s, 1H), 8.35-8.29 (m, 2H), 8.12 (dd,*J*=8.5, 4.2 Hz, 1H), 7.88 (d,*J*=7.6 Hz, 1H), 7.69 (d,*J*=7.5 Hz, 2H), 7.62 (d,*J*=9.5 Hz, 2H), 7.60-7.51 (m, 3H), 7.49 (d,*J*=8.5 Hz, 1H), 7.44-7.35 (m, 7H), 7.32 (q,*J*=7.2 Hz, 2H), 7.25 (t,*J*=7.6 Hz, 6H), 7.14 (t,*J*=7.4 Hz, 3H), 5.76 (s, 1H), 5.19 (s, 2H), 4.39 (q,*J*=7.0, 6.6 Hz, 1H), 4.30-4.17 (m, 2H), 3.73-3.60 (m, 4H), 3.58 (s, 2H), 3.56-3.48 (m, 4H), 3.50-3.37 (m, 16H), 3.37 (tt,*J*=7.4, 3.4 Hz, 4H), 3.33 (s, 7H), 3.28 (s, 1H), 3.19 (d,*J*=6.0 Hz, 5H), 2.01-1.88 (m, 2H), 1.67 (s, 1H), 1.56 (s, 1H), 1.46 (q,*J*=7.3 Hz, 2H), 1.34 (d,*J*=9.6 Hz, 1H), 1.28 (s, 1H), 1.23 (d,*J*=4.1 Hz, 1H).

**D2** (126.2 mg, 0.25 mmol), NMP (4 mL), DIEA (64.6 mg, 0.5 mmol), L3 (340 mg, 0.25 mmol) and HOBT (37.1 mg, 0.27 mmol) were successively added to a 50 mL single-neck flask. The mixture was stirred at room temperature for 12 h and extracted with DCM (50 mL). The organic phase was washed successively with a saturated sodium bicarbonate aqueous solution (50 mL) and water (50 mL), dried over anhydrous sodium sulfate, and concentrated to obtain crude product **L3-D2-1**, which was used directly in the next step; LCMS: [M+1]⁺1727.89 (calculated: 1726.77); ¹H NMR (600 MHz, DMSO-*d*₆) δ 10.14 (s, 1H), 8.13 (s, 1H), 7.86 (s, 2H), 7.81 (s, 1H), 7.67 (d,*J*=7.3 Hz, 2H), 7.60 (s, 1H), 7.53 (s, 2H), 7.45 (d,*J*=8.3 Hz, 1H), 7.42-7.34 (m, 7H), 7.31 (q,*J*=8.8, 7.2 Hz, 3H), 7.24 (t,*J*=7.4 Hz, 7H), 7.13 (t,*J*=7.3 Hz, 3H), 6.49 (s, 1H), 6.28 (s, 1H), 6.12 (s, 1H), 5.42 (s, 2H), 5.28 (s, 2H), 5.06 (s, 2H), 4.60 (s, 1H), 4.39 (d,*J*=8.7 Hz, 1H), 4.29-4.17 (m, 2H), 3.91 (s, 1H), 3.65 (dd,*J*=23.2, 6.3 Hz, 2H), 3.64-3.53 (m, 1H), 3.59 (s, 9H), 3.56-3.46 (m, 3H), 3.47 (d,*J*=3.0 Hz, 1H), 3.46-3.42 (m, 3H), 3.42 (s, 11H), 3.39-3.32 (m, 9H), 3.30 (s, 2H), 3.23-3.15 (m, 5H), 2.53-2.45 (m, 6H), 2.14 (s, 1H), 1.88 (dq,*J*=36.4, 14.0, 7.2 Hz, 3H), 1.67 (s, 1H), 1.56 (d,*J*=10.1 Hz, 1H), 1.47 (s, 2H), 1.28 (s, 1H), 1.23 (d,*J*=4.4 Hz, 1H), 0.87 (t,*J*=7.3 Hz, 3H).

**L3-D2-1** (crude product) and DCM (5 mL) were successively added to a 50 mL single-neck flask and dissolved with stirring. DBU (26.6 mg, 10.6 mmol) was added dropwise, and the mixture was stirred at room temperature for 1 h. The reaction solution was poured into methyl tert-butyl ether (50 mL). The precipitate was collected by filtration and dried to obtain crude product **L3-D2-2**, which was used directly in the next step; LCMS: [M+1]⁺1505.96 (calculated: 1504.71).

Crude product **L3-D2-2** and DCM (10 mL) were successively added to a 50 mL single-neck flask and dissolved with stirring. The prepared Fmoc-NH-PEG2-CH2CH2COOSu (124 mg, 0.25 mmol) was subsequently added, and the mixture was stirred at room temperature for 2 h. The reaction solution was concentrated and purified by silica gel column chromatography to obtain **L3-D2-3** (87 mg, total yield over three steps 18%); LCMS: [M+1]⁺1886.99 (calculated: 1885.86); ¹H NMR (600 MHz, DMSO-*d*₆) δ 10.10 (s, 1H), 8.13 (t,*J*=6.2 Hz, 2H), 7.90-7.82 (m, 2H), 7.67 (d,*J*=7.6 Hz, 2H), 7.62 (d,*J*=10.0 Hz, 2H), 7.47 (q,*J*=8.5, 6.2 Hz, 1H), 7.38 (dd,*J*=10.8, 7.6 Hz, 7H), 7.31 (dt,*J*=17.4, 6.8 Hz, 3H), 7.30-7.20 (m, 7H), 7.15 (q,*J*=7.3, 6.8 Hz, 3H), 6.49 (s, 1H), 6.28 (t,*J*=3.2 Hz, 1H), 6.13 (s, 1H), 5.42 (d,*J*=2.7 Hz, 2H), 5.24 (d,*J*=16.8 Hz, 2H), 5.07 (s, 2H), 4.58 (d,*J*=28.1 Hz, 1H), 4.37 (q,*J*=7.4 Hz, 1H), 4.27 (d,*J*=6.9 Hz, 2H), 4.18 (q,*J*=8.0, 6.9 Hz, 1H), 3.92 (d,*J*=12.5 Hz, 1H), 3.74 (d,*J*=5.6 Hz, 2H), 3.72-3.54 (m, 9H), 3.54-3.46 (m, 2H), 3.51-3.41 (m, 21H), 3.38 (d,*J*=5.0 Hz, 3H), 3.39-3.31 (m, 12H), 3.31 (d,*J*=17.6 Hz, 4H), 3.27 (s, 1H), 3.19 (s, 1H), 3.11 (q,*J*=6.1 Hz, 2H), 2.59 (s, 1H), 2.37 (t,*J*=6.5 Hz, 2H), 2.15 (d,*J*=14.1 Hz, 1H), 1.97-1.78 (m, 4H), 1.68 (t,*J*=9.0 Hz, 1H), 1.60-1.53 (m, 1H), 1.48 (p,*J*=7.4 Hz, 2H), 1.35 (s, 1H), 1.30-1.20 (m, 3H), 0.91-0.81 (m, 3H).

**L3-D2-3** (87 mg, 0.046 mmol) and DCM (5 mL) were successively added to a 50 mL single-neck flask and partially dissolved with stirring. DBU (4.9 mg) was added dropwise. Upon completion of the addition, the mixture was stirred at room temperature for 30 min. The reaction solution was poured into methyl tert-butyl ether (60 mL) and centrifuged, and the supernatant was discarded. The crude product was purified by silica gel column chromatography to obtain product **L3-D2-4** (70 mg, yield 91%, HPLC purity 96%); LCMS: [M+1]⁺ 1664.78 (calculated: 1663.79); ¹H NMR (600 MHz, DMSO) δ 10.10 (s, 1H), 8.25-8.05 (m, 2H), 7.65-7.58 (m, 2H), 7.55-7.45 (m, 2H), 7.40-7.33 (m, 6H), 7.35-7.20 (m, 8H), 7.18-7.10 (m, 3H), 6.65-6.05 (m, 3H), 5.50-4.90 (m, 6H), 4.86-4.33 (m, 3H), 3.67-3.26 (m, 61H), 2.40-2.30 (m, 2H), 1.97-1.31 (m, 11H), 0.90-0.84 (m, 3H).

**L3-D2-4** (70 mg, 0.042 mmol) and DCM (5 mL) were successively added to a 50 mL single-neck flask and dissolved with stirring. Bromoacetic anhydride (10.9 mg, 0.042 mmol) was added. Upon completion of the addition, the mixture was stirred at room temperature for 30 min. The reaction solution was concentrated and purified by silica gel column chromatography to obtain **L3-D2-5** (60 mg, yield 80%, HPLC 98%); LCMS: [M+1]⁺1784.93 (calculated: 1783.72).

**L3-D2-5** (60 mg, 33.6 µmol) and DCM (3 mL) were added to a 50 mL single-neck flask and dissolved with stirring. TFA (0.3 mL) was added dropwise, and the mixture was stirred at room temperature for 1 h. The reaction solution was added dropwise into methyl tert-butyl ether (30 mL), followed by centrifugation. The supernatant was discarded, and the residual solid was dried to obtain yellow solid product **L3-D2** (35 mg, yield 67%, HPLC 97%); LCMS: [M+1]⁺1542.84 (calculated: 1541.61); ¹H NMR (600 MHz, DMSO-*d*₆) δ 10.12 (s, 1H), 8.19 (t,*J*=5.8 Hz, 2H), 7.66 (dt,*J*=11.8, 5.8 Hz, 4H), 7.46 (d,*J*=8.5 Hz, 1H), 7.23 (s, 1H), 6.29 (s, 1H), 6.16 (s, 1H), 5.42 (s, 2H), 5.29 (s, 2H), 5.06 (s, 2H), 4.43-4.37 (m, 1H), 3.91 (d,*J*=7.3 Hz, 1H), 3.85 (s, 1H), 3.78 (s, 1H), 3.79-3.69 (m, 2H), 3.71 (s, 1H), 3.61 (dq,*J*=13.2, 7.3 Hz, 6H), 3.58-3.50 (m, 2H), 3.50-3.36 (m, 23H), 3.40-3.34 (m, 4H), 3.28-3.20 (m, 1H), 3.19 (d,*J*=4.1 Hz, 6H), 2.77 (h,*J*=6.4 Hz, 2H), 2.40 (t,*J*=6.5 Hz, 2H), 2.15 (s, 1H), 1.86 (dp,*J*=21.4, 7.2 Hz, 3H), 1.76 (qd,*J*=10.5, 7.0, 5.3 Hz, 1H), 1.68-1.57 (m, 1H), 1.54 (hept,*J*=6.7 Hz, 2H), 1.43-1.27 (m, 2H), 0.87 (t,*J*=7.3 Hz, 3H); ¹³C NMR (151 MHz, DMSO) δ 173.04, 172.28, 171.25, 158.70, 158.47, 150.55, 146.77, 128.56, 118.58, 96.41, 72.87, 71.70, 70.25, 70.20, 70.11, 70.01, 69.91, 69.37, 69.17, 68.31, 67.14, 65.71, 61.82, 58.48, 50.01, 42.58, 38.32, 36.30, 30.66, 29.92, 27.14, 22.83, 8.23.

### 3.2 L3-D2-Ab_{nectin-4}antibody-drug conjugate (ADC3)

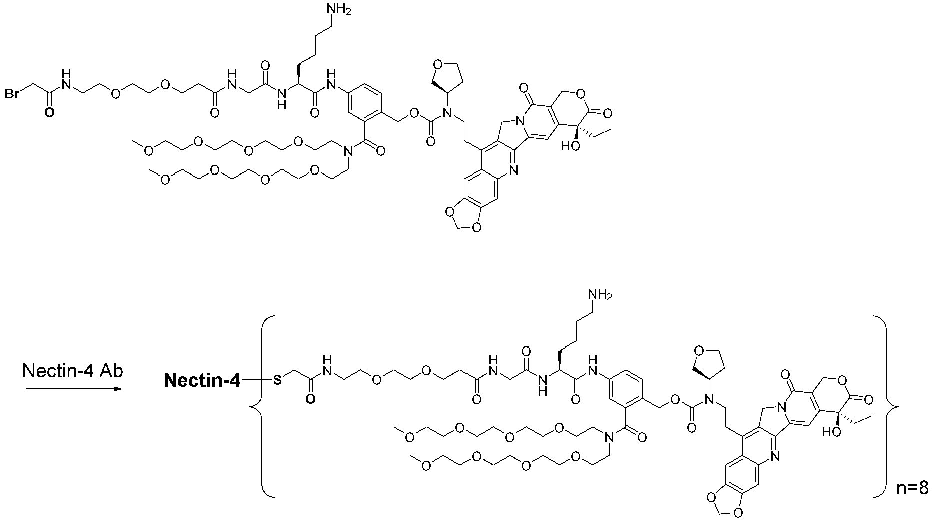

The Nectin4 antibody (10.0 mg/mL, 10 mg, 0.066 mmol) was taken, and its pH was adjusted to 7.2 with a 1 M disodium hydrogen phosphate solution. Subsequently, a 0.1 M EDTA disodium solution (25 µL) and a freshly prepared TCEP·HCl solution (10 mM, 0.04 mL) were added sequentially. The mixture was incubated on a rotating disk at 25°C for 3 h.

Compound **L3-D2** (1.23 mg, 0.80 mmol) was dissolved in 0.12 mL of DMA. The resulting solution was added to the above solution system and mixed thoroughly. The mixture was incubated on a rotating disk at room temperature for 16 h. Upon completion of the reaction, small molecules were removed, and the buffer was exchanged into a 20 mM PB solution (pH 6.3) using a NAP-5 gel column (Cytiva) to afford the antibody-drug conjugate **ADC3** (3.2 mg/mL, 2 mL).

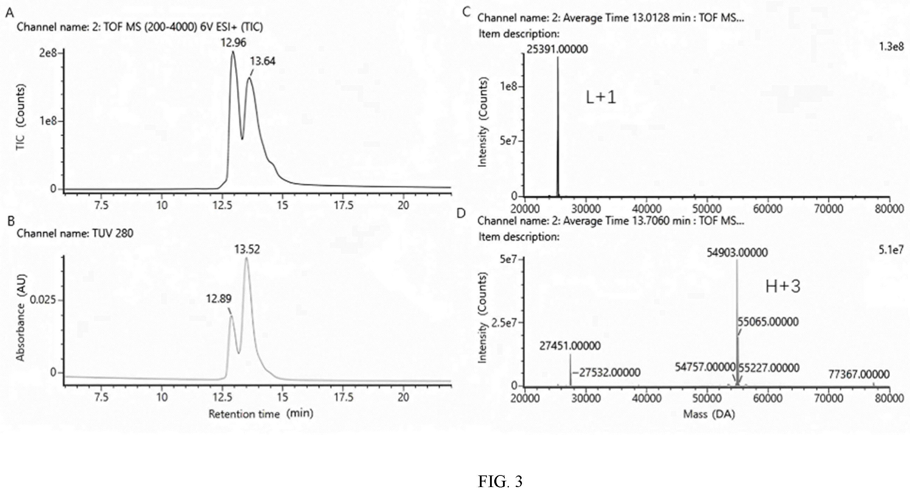

RP-MS calculated: average: n=7.9; MS results showed that the antibody light chain (L) was linked to one **L3-D2** (linker-payload), and the heavy chain (H) was linked to three **L3-D2** (linker-payload) (FIG. 3).

### Example 4: Ab_{nectin-4}-(S-7-(N-(acetamido-PEG₂-propionyl-Gly-Lys-PAB(3-bis-PEG₄-carbonyl)C)-N-(4-tetrahydropyranyl))aminoethyl camptothecin)₈ (ADC4)

### 4.1 7-(N-(bromoacetamide-PEG₂-propionyl-Gly-Lys-PAB(3-bis-PEG₄-carbonyl)C)-N-(4-tetrahydropyranyl))aminoethyl camptothecin (L3-D3)

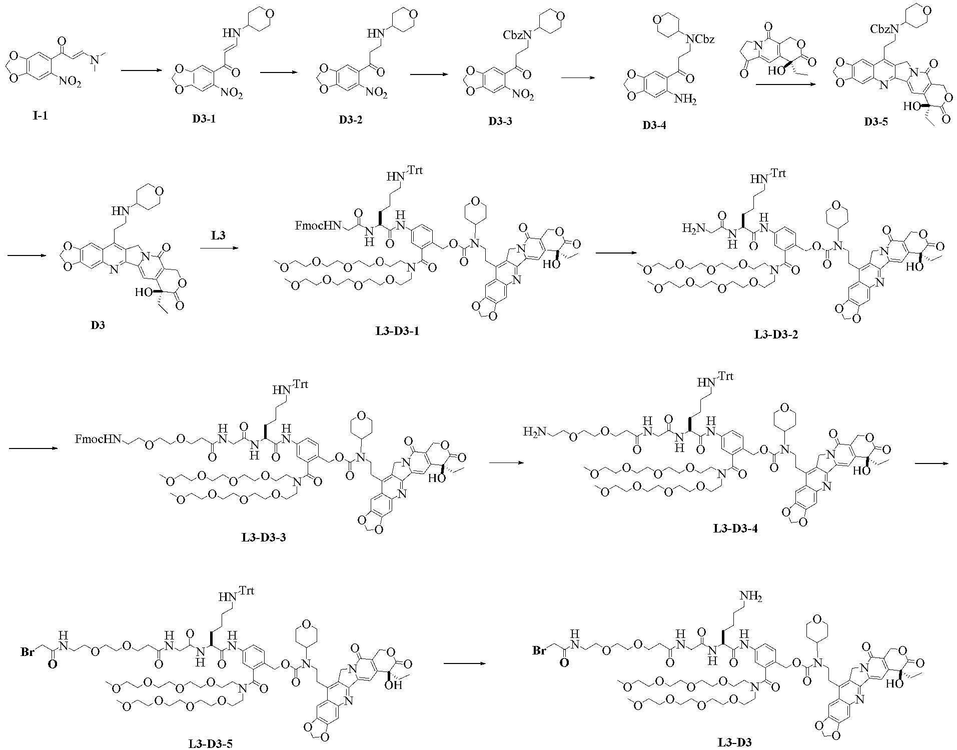

Intermediate **I-1** (18 g, 68.12 mmol) was added to IPA (180 mL), and the reation solution was stirred. DIEA (17.61 g, 136.24 mmol) and 4-aminotetrahydropyran hydrochloride (37.50 g, 272.48 mmol) were then added. The mixture was heated under reflux for 12 h. The reaction solution was concentrated until the residual volume of IPA reached approximately 20 mL. Water (200 mL) was added, and the resulting reaction solution was stirred for 2 h. The solid was collected by filtration and dried at 40°C to obtain product **D3-1** (20.2 g, yield 91.7%); LCMS: [M+1]⁺320.98 (calculated: 320.10).

Compound **D3-1** (16.9 g, 52.63 mmol) was added to DCM (310 mL), followed by the addition of acetic acid (63.05 g, 1.05 mol). The mixture was stirred until all solids were completely dissolved. The internal temperature of the reaction solution was maintained at 15°C, and sodium triacetoxyborohydride (27.89 g, 131.56 mmol) was added in four portions of approximately 7 g each at an interval of about 5 min. The internal temperature of the reaction solution was maintained at 15°C for 2 h. The reaction solution was poured into an ice-cold sodium carbonate aqueous solution (sodium carbonate: 130 g, and ice water: 1L), the internal temperature was controlled at 15°C. The reaction solution was srirred and subjected to phase separation. The organic phase was washed with water and dried over anhydrous sodium sulfate to obtain a DCM solution of compound **D3-2**. The solution was used directly in the next step without further purification.

Under ice cooling and nitrogen protection, DIEA (13.58 g, 105.26 mmol) was added to the above DCM solution of D3-2. Cbz-Cl (10.11 g, 59.27 mmol) was then added dropwise. The mixture was further stirred in an ice bath for 12 h. The organic phase was washed twice with a 10% citric acid aqueous solution (200 mL each time), followed by sequential washing with a saturated sodium bicarbonate aqueous solution (200 mL) and saturated brine (200 mL). The organic phase was dried over anhydrous sodium sulfate and concentrated to obtain compound **D3-3** (22.9 g, yield 95% over two steps); LCMS: [M+1]⁺457.28 (calculated: 456.15).

Compound **D3-3** (22.7 g, 49.7 mmol) was added to a 1 L single-neck flask, followed by the addition of DMF (50 mL). Under ice cooling and nitrogen protection, the freshly prepared reducing solution (H₂O (250 mL)/sodium dithionite (39.30 g, 225.72 mmol)/sodium carbonate (19.14 g, 180.58 mmol)) was slowly added dropwise. Upon completion of the addition, the reaction mixture was gradually heated to 40°C and stirred for 2 h. The reaction solution was filtered, and the filtrate was concentrated. Ethyl acetate (500 mL) and water (500 mL) were added for extraction and liquid separation. The organic phase was dried over anhydrous sodium sulfate and concentrated. The crude product was purified by silica gel column chromatography to obtain yellow oil **D3-4** (20.3 g, yield 95.7%, HPLC 98%); LCMS: [M+1]⁺427.22 (calculated:426.19).

Compound **D3-4** (18.56 g, 43.55 mmol), (S)-4-ethyl-4-hydroxy-7,8-dihydro-1H-pyrano[3,4-f]indolizine-3,6,10(4H)-trione (13.75 g, 52.27 mmol) and PPTS (218.88 g, 871.00 mmol) were successively added to a 500 mL single-neck flask. The mixture was reacted at 110 °C for 12 h under argon protection. The reaction solution was poured into methanol (1000 mL) and stirred for 2 h. A dark brown solid was collected by filtration, which was then washed with methanol (300 mL) for another 2 h. A light brown solid was collected by filtration. The solid was dried at 40°C to obtain compound **D3-5** (11.0 g, yield 38.7%, HPLC 97%); LCMS: [M+1]⁺653.98 (calculated: 653.24);¹H NMR (500 MHz, DMSO-d₆) δ 7.38 (t,*J* = 68.6 Hz, 7H), 6.49 (s, 1H), 6.29 (s, 2H), 5.43 (s, 2H), 5.26 (dd,*J* = 55.5, 36.0 Hz, 4H), 4.05 (s, 1H), 3.90 (d,*J* = 7.6 Hz, 2H), 3.46 (d,*J* = 8.4 Hz, 2H), 1.93-1.74 (m, 4H), 1.57 (s, 2H), 0.88 (t,*J* = 7.3 Hz, 3H).

Compound **D3-5** (10.32 g, 15.78 mmol), dichloromethane (2.5 L), methanol (2.5 L) and 10% Pd/C (15 g) were added into a 10 L single-neck flask. The mixture was reacted at room temperature under a hydrogen balloon atmosphere for 20 h. The Pd/C catalyst was removed by filtration, and the reaction solution was evaporated to dryness. The residue was stirred with 30 mL of dichloromethane/methanol mixed solution (V/V = 1:1) for 24 h. After filtration, the solid was dried at 40°C for 3 h to obtain product **D3** (6.1 g, yield 74%, HPLC 95.0%); LCMS: [M+1]⁺520.22 (calculated: 519.20); ¹H NMR (500 MHz, DMSO-d₆) δ 7.59 (s, 1H), 7.47-7.41 (m, 1H), 7.25-7.14 (m, 1H), 6.54 (s, 1H), 6.27 (t,*J*=9.9 Hz, 2H), 5.77 (s, 1H), 5.44 (d,*J*=16.5 Hz, 2H), 5.23-5.11 (m, 2H), 3.83 (d,*J*=9.5 Hz, 2H), 3.23 (t,*J*=24.9 Hz, 4H), 2.93 (d,*J*=32.7 Hz, 3H), 1.99-1.72 (m, 4H), 1.33 (s, 2H), 0.96-0.81 (m, 3H).

**D3** (130.0 mg, 0.25 mmol), N-methyl-2-pyrrolidone (NMP) (4 mL), DIEA (64.6 mg, 0.5 mmol), **L3** (340 mg, 0.25 mmol) and HOBT (37.1 mg, 0.27 mmol) were successively added to a 50 mL single-neck flask. The mixture was stirred at room temperature for 12 h and extracted with DCM. The organic phase was washed successively with a saturated sodium bicarbonate aqueous solution (50 mL) and water (50 mL), dried over anhydrous sodium sulfate, and concentrated to obtain **L3-D3-1** (crude product), which was used directly in the next step; LCMS: [M+1]⁺1741.93 (calculated: 1740.79).

The above L3-D3-1 (crude product) and DCM (5 mL) were successively added to a 50 mL single-neck flask and dissolved with stirring. DBU (26.6 mg) was added dropwise, and the mixture was stirred at room temperature for 1 h. The reaction solution was poured into methyl tert-butyl ether (50 mL) and centrifuged. The resulting solid was dried to obtain **L3-D3-2** (crude product), which was used directly in the next step without further purification; LCMS: [M+1]⁺1519.96 (calculated: 1518.72).

The above L3-D3-2 (crude product) and DCM (10 mL) were successively added to a 50 mL single-neck flask and dissolved with stirring. The prepared Fmoc-NH-PEG2-CH2CH2COOSu (124 mg, 0.25 mmol) was added, and the mixture was further stirred at room temperature for 2 h. The reaction solution was concentrated and purified by silica gel column chromatography to obtain product **L3-D3-3** (89 mg, 18.7% overall yield over three steps); LCMS: [M+1]⁺1900.97 (calculated: 1899.88); ¹H NMR (600 MHz, DMSO-*d*₆) δ 10.09 (s, 1H), 8.12 (s, 2H), 7.87 (dd,*J*=15.9, 7.5 Hz, 3H), 7.68 (dd,*J*=12.8, 7.5 Hz, 2H), 7.35-7.22 (m, 9H), 6.29 (s, 1H), 6.11 (s, 1H), 5.42 (s, 2H), 5.33 (d,*J*=40.1 Hz, 2H), 5.03 (s, 1H), 4.28 (dd,*J*=11.9, 6.9 Hz, 2H), 3.95-3.85 (m, 2H), 3.74 (d,*J*=5.6 Hz, 2H), 3.58 (h,*J*=9.2, 7.8 Hz, 3H), 3.54-3.46 (m, 3H), 3.44 (q,*J*=10.7, 8.8 Hz, 22H), 3.37 (tt,*J*=6.3, 4.3, 3.5 Hz, 6H), 1.84 (ddd,*J*=37.1, 19.5, 10.2 Hz, 2H), 1.40-1.22 (m, 3H), 0.86 (dt,*J*=13.0, 7.2 Hz, 4H).

**L3-D3-3** (87 mg, 0.045 mmol) and DCM (6 mL) were successively added to a 50 mL single-neck flask and dissolved with stirring. DBU (4.9 mg) was added dropwise, and the mixture was stirred at room temperature for 30 min. The resulting reaction solution was poured into methyl tert-butyl ether (50 mL) and centrifuged, and the supernatant was discarded. The crude product was purified by silica gel column chromatography to obtain **L3-D3-4** (74 mg, yield 97%, HPLC purity 96%); LCMS: [M+1]⁺1678.96 (calculated: 1678.82).

**L3-D3-4** (73 mg, 0.043 mmol) and DCM (5 mL) were successively added to a 50 mL single-neck flask and dissolved with stirring. Bromoacetic anhydride (11.0 mg, 0.043 mmol) was added, and the mixture was stirred at room temperature for 30 min. The reaction solution was concentrated and purified by silica gel column chromatography to obtain **L3-D3-5** (65 mg, yield 84%, HPLC 98%); LCMS: [M+1]⁺1798.98 (calculated: 1798.74).

**L3-D3-5** (64 mg, 35.6 µmol) and DCM (5 mL) were added to a 50 mL single-neck flask and dissolved with stirring. TFA (0.3 mL) was then added dropwise to the reaction solution, which was stirred at room temperature for 1 h. The reaction solution was added dropwise into methyl tert-butyl ether (30 mL). The solid was collected by centrifugation and dried to obtain yellow solid product **L3-D3** (35 mg, yield 63%, HPLC 97%); LCMS: [M+1]⁺1556.87 (calculated: 1556.63); ¹H NMR (500 MHz, DMSO-*d*₆) δ 10.12 (s, 1H), 8.40-8.15 (m, 2H), 7.75-7.65 (m, 3H), 7.5-7.40 (m, 2H), 7.24 (s, 1H), 6.35-6.10 (m, 2H), 5.43 (s, 2H), 5.40-5.25 (m, 2H), 5.15-5.00 (m, 2H), 4.45-4.30 (m, 2H), 4.08-3.20 (m, 63H), 2.85-2.70 (m, 2H), 2.40 (t,*J*=6.2 Hz, 2H), 1.95-1.70 (m, 5H), 1.67-1.46 (m, 5H), 1.45-1.27 (m, 2H), 0.88 (t,*J*=7.2 Hz, 3H); ¹³C NMR (126 MHz, DMSO-*d*₆) δ 173.00, 171.22, 166.56, 158.71, 158.44, 157.29, 151.37, 150.59, 149.88, 146.81, 128.62, 118.54, 103.07, 72.88, 71.71, 70.26, 70.21, 70.13, 70.02, 69.96, 69.91, 69.17, 67.13, 65.71, 65.37, 58.48, 50.16, 48.86, 44.46, 42.61, 39.15, 36.32, 31.77, 30.72, 29.91, 27.15, 22.84, 15.63, 8.23.

### 4.2 L3-D3-Ab_{nectin-4} antibody-drug conjugate (ADC4)

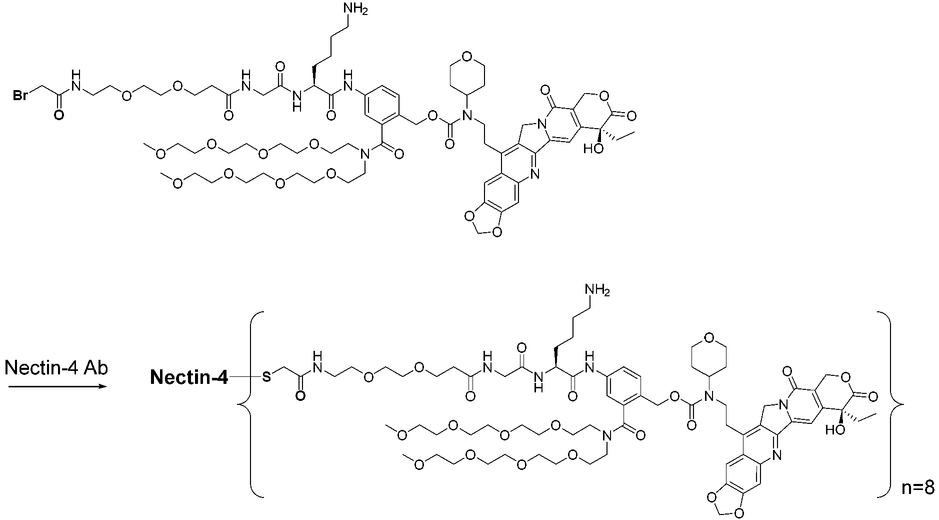

The Nectin4 antibody (10.0 mg/mL, 10 mg, 0.066 mmol) was taken, and its pH was adjusted to 7.2 with a 1 M disodium hydrogen phosphate solution. Subsequently, a 0.1 M disodium ethylenediaminetetraacetate solution (25 µL) and a freshly prepared TCEP·HCl solution (10 mM, 0.04 mL) were added sequentially. The mixture was incubated on a rotator at 25°C for 3 h.

Compound **L3-D3** (1.25 mg, 0.80 mmol) was dissolved in 0.13 mL of DMA. The resulting solution was added to the above reaction system and mixed thoroughly. The mixture was incubated on a rotator at room temperature for 16 h. Upon completion of the reaction, small molecules were removed and the buffer was exchanged to a 20 mM PB solution (pH 6.3) via a NAP-5 gel column (Cytiva) to obtain the antibody-drug conjugate ADC4 (3.2 mg/mL, 2 mL).

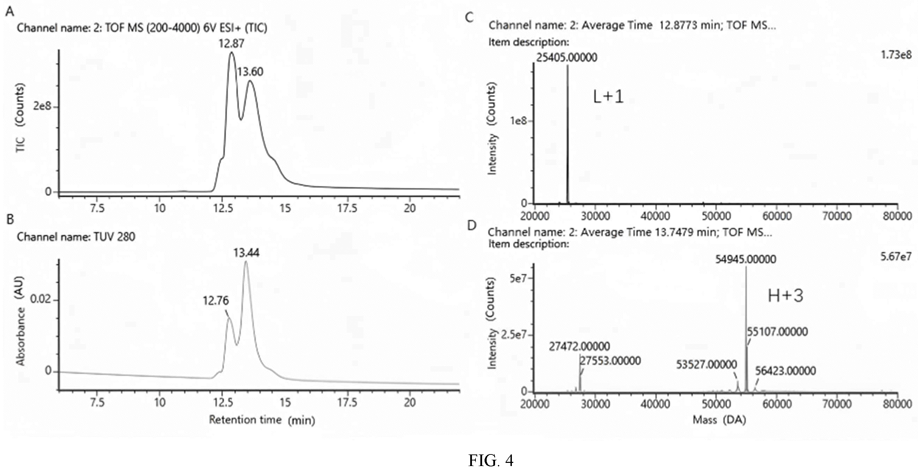

RP-MS calculated: average: n=7.8; MS results showed that the antibody light chain (L) was linked to one **L3-D3** (linker-payload), and the heavy chain (H) was linked to three **L3-D3** (linker-payload) (FIG. 4).

### Example 5: Ab_{nectin-4}-(S-7-(N-(bromoacetamide-PEG₂-propionyl-Gly-Lys-PAB(2-PEG₈-oxy)C)-N-((R)-3-tetrahydrofuranyl))aminoethyl camptothecin)s (ADC5)

### 5.1 7-(N-(bromoacetamide-PEG₂-propionyl-Gly-Lys-PAB(2-PEG₈-oxy)C)-N-((R)-3-tetrahydrofuranyl))aminoethyl camptothecin (L4-D2)

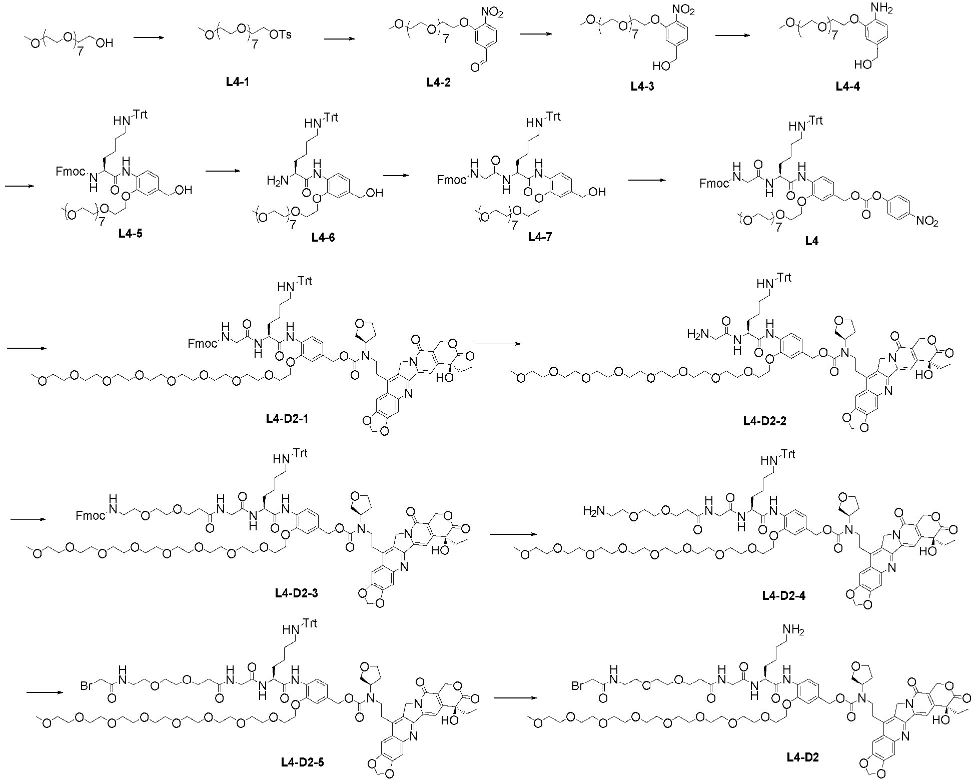

2-[2-[2-[2-[2-[2-[2-(2-methoxyethoxy)ethoxy]ethoxy]ethoxy]ethoxy]ethoxy] ethoxy]ethanol (50 g, 130.05 mmol), DCM (500 mL), p-TsCl (29.75 g, 156.06 mmol) and TEA (39.48 g, 390.15 mmol) were added to a reaction flask. The mixture was stirred at room temperature for 16 h. The reaction solution was concentrated under reduced pressure. The residue was extracted with ethyl acetate. The organic phase was washed with a saturated sodium chloride aqueous solution, dried over anhydrous sodium sulfate and concentrated. The crude product was purified by silica gel column chromatography to obtain colorless liquid tosylate intermediate **L4-1** (50 g, yield 71.37 %); LCMS: [M+H]⁺539.38 (calculated: 538.24).

3-hydroxy-4-nitrobenzaldehyde (23.27 g, 139.24 mmol), L4-1 (50 g, 92.82 mmol), DMF (300 mL) and potassium carbonate (38.49 g, 278.47 mmol) were added to a reaction flask. The mixture was stirred at 70°C for 2 h and concentrated. The residue was extracted with ethyl acetate. The organic phase was washed with water and dried over anhydrous sodium sulfate. The crude product was purified by silica gel column chromatography to obtain intermediate **L4-2** as a brown liquid (27 g, yield 54.51%); LCMS: [M+H]⁺534.38 (calculated: 533.25).

**L4-2** (21 g, 39.36 mmol), methanol (200 mL) and sodium borohydride (2.23 g, 59.04 mmol) were added to a reaction flask. The mixture was stirred at room temperature for 4 h and concentrated under reduced pressure. The residue was extracted with ethyl acetate. The organic phase was washed with saturated brine and dried over anhydrous sodium sulfate. The mixture was filtered, and the filtrate was concentrated to obtain brown liquid product **L4-3** (19 g, yield 90.12%).

**L4-3** (19 g, 35.47 mmol), MeOH (250 mL), 10% Pd/C (2 g, 7.1 mmol) and DIPEA (9.17 g, 70.95 mmol) were added to a reaction flask. The reaction mixture was stirred under a hydrogen balloon atmosphere for 48 h. The reaction solution was filtered and concentrated under pressure to obtain brown liquid compound **L4-4** (17.5 g, yield 97.58%); LCMS: [M+H]⁺506.21 (calculated: 505.29).

**L4-4** (10 g, 19.78 mmol), Fmoc-Lys(Trt)OH (12.08 g, 19.78 mmol), DCM (300 mL), EDCI (3.98 g, 20.77 mmol), HOBT (2.81 g, 20.77 mmol) and DIPEA (3.83 g, 29.67 mmol) were added to a 1000 mL single-neck flask. The mixture was stirred at 25°C for 2 h. a saturated sodium bicarbonate aqueous solution (300 mL) was added, and the mixture was stirred and subjected to extraction and liquid separation. The organic phase was sequentially washed with a citric acid aqueous solution and saturated brine and dried over anhydrous magnesium sulfate. The mixture was filtered, and the filtrate was concentrated under reduced pressure to obtain Fmoc-Lys(Trt)PAB(PEG8) **L4-5** (17 g, yield 75.13 %); LCMS: [M+1]⁺1098.21 (calculated: 1097.34).

**L4-5** (17 g, 14.86 mmol), DCM (400 mL) and DBU (1.13 g, 7.43 mmol) were added into a 1000 mL single-neck flask. The mixture was stirred at 25°C for 1 h and concentrated to obtain **L4-6**, which was used directly in the next reaction; LCMS: [M+1]⁺876.34 (calculated: 875.10).
HOBT (4.18 g, 30.96 mmol), Fmoc-Gly-OH (4.83 g, 16.25 mmol), EDCI (3.12 g, 16.25 mmol) and DIPEA (3.00 g, 23.22 mmol) were added to the reaction solution obtained from the previous step. The mixture was stirred at 25 °C for 2 h. A citric acid aqueous solution (200 mL) was added, and the mixture was stirred, extracted and separated. The organic phase was successively washed with a saturated sodium bicarbonate aqueous solution and saturated brine, separated, dried over anhydrous magnesium sulfate and filtered. The filtrate was concentrated and purified by silica gel column chromatography to obtain Fmoc-Gly-Lys(Trt)PAB(PEG8) **L4-7** (15 g, yield 83.88% over two steps); LCMS: [M+1]⁺1155.60 (calculated: 1154.40); ¹H NMR (600 MHz, DMSO-d₆) δ 8.98 (s, 1H), 8.16 (d,*J*=7.7 Hz, 1H), 7.87 (dd,*J*=16.8, 7.8 Hz, 2H), 7.68 (d,*J*=7.5 Hz, 2H), 7.53 (t,*J*=6.2 Hz, 1H), 7.40 (dd,*J*=20.1, 7.7 Hz, 6H), 7.32 (q,*J*=7.2 Hz, 2H), 7.26 (t,*J*=7.6 Hz, 5H), 7.15 (t,*J*=7.3 Hz, 3H), 7.01 (s, 1H), 6.86 (d,*J*=8.2 Hz, 1H), 5.76 (s, 3H), 5.15 (t,*J*=5.7 Hz, 1H), 4.44 (d,*J*=5.8 Hz, 3H), 4.27-4.18 (m, 2H), 4.10 (t,*J*=5.0 Hz, 2H), 3.79-3.64 (m, 4H), 3.57 (dd,*J*=5.9, 3.7 Hz, 2H), 3.52-3.43 (m, 22H), 3.41 (dd,*J*=5.8, 3.8 Hz, 2H), 3.34 (s, 3H), 2.50 (d,*J*=4.0 Hz, 2H), 1.94 (q,*J*=7.4 Hz, 2H), 1.72 (ddt,*J*=15.6, 11.0, 5.4 Hz, 1H), 1.56 (tq,*J*=13.5, 8.4, 6.5 Hz, 1H), 1.46 (hept,*J*=7.9, 7.4 Hz, 2H), 1.35-1.27 (m, 2H).

A 250 mL single-neck flask was used at room temperature. **D2** (0.5 g, 918.08 µmol), NMP (10 mL), **L4** (1.21 g, 891.68 µmol), HOBT (140.59 mg, 918.08 µmol) and DIPEA (118.65 mg, 918.08 µmol, 159.91 µL) were successively added to the flask. The mixture was stirred at room temperature for 16 h. Diethyl ether (100 mL) was added, and the precipitated solid was collected by filtration to obtain pale yellow solid **L4-D2-1** (1.5 g, yield 87.1%); LCMS: [M+1]⁺1687.56 (calculated: 1686.92).

**L4-D2-1** (1.5 g, 799.54 µmol), NMP (9 mL) and piperidine (824.84 mg, 9.69 mmol, 956.90 µL) were successively added to a 250 mL single-neck flask at room temperature. The mixture was stirred at room temperature for 1 h. Diethyl ether (100 mL) was added, and the precipitated solid was collected by filtration to obtain light brown solid L4-D2-2 (1.2 g, yield 77.0%); LCMS: [M+1]⁺1465.38 (calculated: 1464.67).

**L4-D2-2** (1.2 g, 678.31 µmol), DMF (10 mL), Fmoc-PEG2-OSu (396.22 mg, 739.75 µmol) and DIPEA (103.14 mg, 798.01 µmol, 139.00 µL) were successively added to a 100 mL single-neck flask at room temperature. The mixture was stirred at room temperature for 0.5 h. Silica gel powder (10 g) was added for sample loading, and the mixture was purified by silica gel column chromatography to obtain off-white solid **L4-D2-3** (410 mg, yield 25.9%); LCMS: [M+1]⁺1847.01 (calculated: 1846.10); 1H NMR (600 MHz, DMSO-d6) δ 10.54 (s, 2H), 8.15 (d, J = 0.6 Hz, 1H), 8.08 (t, J = 5.8 Hz, 1H), 7.95 (d, J = 7.8 Hz, 1H), 7.86 (d, J = 7.6 Hz, 2H), 7.67 (d, J = 7.5 Hz, 2H), 7.39 (dd, J = 10.9, 7.6 Hz, 8H), 7.31 (q, J = 7.5, 6.7 Hz, 3H), 7.28-7.22 (m, 7H), 7.15 (q, J = 8.9, 7.3 Hz, 4H), 6.49 (s, 1H), 6.28 (s, 1H), 6.22 (s, 1H), 5.42 (d, J = 2.4 Hz, 2H), 5.26 (s, 2H), 5.14 (s, 2H), 4.60 (d, J = 7.8 Hz, 1H), 4.44 (q, J = 7.3 Hz, 1H), 4.27 (d, J = 7.0 Hz, 2H), 4.13 (s, 1H), 3.94 (s, 1H), 3.84-3.69 (m, 5H), 3.67-3.63 (m, 1H), 3.57 (t, J = 6.5 Hz, 2H), 3.51-3.44 (m, 26H), 3.42-3.35 (m, 4H), 3.29 (s, 1H), 3.21 (s, 3H), 3.11 (q, J = 5.9 Hz, 2H), 2.59 (s, 8H), 2.36 (t, J = 6.5 Hz, 2H), 2.15 (dt, J = 13.3, 8.5, 5.0 Hz, 1H), 1.96-1.88 (m, 2H), 1.85 (dq, J = 14.0, 7.1 Hz, 3H), 1.70 (s, 1H), 1.55 (d, J = 9.1 Hz, 1H), 1.46 (q, J = 7.5 Hz, 2H), 1.31-1.22 (m, 4H), 0.87 (t, J = 7.3 Hz, 3H).

**L4-D2-3** (0.41 g, 217.49 µmol), DMF (4 mL) and piperidine (370.37 mg, 4.35 mmol, 429.66 µL) were successively added to a 10 mL single-neck round-bottom flask at room temperature. The mixture was stirred at room temperature for 1 h. Silica gel powder (5 g) was added for sample loading, and the resulting mixture was purified by silica gel column chromatography to obtain light yellow solid L4-D2-4 (230 mg, yield 61.05%); LCMS: [M+1]⁺1624.75 (calculated: value: 1623.86); ¹H NMR (600 MHz, DMSO-d₆) δ 7.96 (s, 3H), 7.51-7.45 (m, 1H), 7.39 (d,*J*=7.9 Hz, 5H), 7.29-7.21 (m, 6H), 7.16 (q,*J*=7.4 Hz, 3H), 7.02 (s, 1H), 6.51 (s, 1H), 6.27 (s, 1H), 6.23 (s, 1H), 5.44-5.41 (m, 1H), 5.24 (s, 1H), 5.15 (s, 2H), 4.62 (s, 1H), 4.44 (q,*J*=7.2, 6.8 Hz, 1H), 4.13-4.09 (m, 1H), 3.79 (qd,*J*=16.7, 5.8 Hz, 1H), 3.73 (s, 1H), 3.60 (td,*J*=5.9, 5.3, 2.9 Hz, 3H), 3.57-3.45 (m, 21H), 3.41 (dd,*J*=5.9, 3.7 Hz, 2H), 3.38 (s, 20H), 3.30 (s, 1H), 3.22 (s, 2H), 2.94 (t,*J*=5.3 Hz, 1H), 2.89 (s, 5H), 2.73 (s, 5H), 2.39 (t,*J*=6.5 Hz, 1H), 2.16 (ddt,*J*=12.4, 7.7, 4.5 Hz, 1H), 1.95 (t,*J*=6.5 Hz, 2H), 1.87 (ddd,*J*=28.8, 13.7, 6.8 Hz, 2H), 1.75-1.66 (m, 1H), 1.63-1.54 (m, 1H), 1.51-1.45 (m, 2H), 1.30 (s, 1H), 1.27-1.21 (m, 4H), 0.87 (dt,*J*=20.0, 7.2 Hz, 3H).

**L4-D2-4** (100 mg, 60.13 µmol), DMF (2 mL) and bromoacetic anhydride (18 mg, 60.13 µmol) were successively added to a 10 mL single-neck flask at room temperature. The mixture was stirred at room temperature for 2 h. Silica gel powder (3 g) was added for sample loading, and the mixture was purified by silica gel column chromatography to obtain light yellow solid **L4-D2-5** (80 mg, yield 72.34%); LCMS: [M+1]⁺1745.62 (calculated: 1744.79).

**L4-D2-5** (80 mg, 44.85 µmol), DCM (1 mL) and TFA (153.41 mg, 1.35 mmol, 102.96 µL) were successively added to a 10 mL single-neck round-bottom flask at room temperature. The mixture was stirred at room temperature for 2 h. Diethyl ether (10 mL) was added, and the reaction solution was centrifuged at 10000 rpm for 5 min. The supernatant was discarded, and the residual solvent was concentrated under reduced pressure to obtain yellow solid powder **L4-D2** (50 mg, yield 70.15%); LCMS: [M+1]⁺1503.33 (calculated: 1502.47); ¹H NMR (600 MHz, DMSO-d₆) δ 9.05 (s, 1H), 8.33 (t,*J*=5.7 Hz, 1H), 8.24 (dd,*J*=7.9, 4.3 Hz, 1H), 8.14 (t,*J*=5.7 Hz, 1H), 7.97 (d,*J*=8.2 Hz, 1H), 7.80 (s, 1H), 7.66 (q,*J*=6.2 Hz, 4H), 7.52-7.48 (m, 1H), 7.37 (s, 1H), 7.24 (s, 1H), 7.18 (s, 1H), 7.02 (d,*J*=15.5 Hz, 1H), 6.49 (s, 1H), 6.28 (s, 1H), 6.25 (s, 1H), 5.43 (s, 2H), 5.28 (s, 2H), 5.14 (s, 2H), 4.61 (d, J=7.6 Hz, 1H), 4.48 (t,*J*=5.8 Hz, 1H), 4.18 (d,*J*=8.7 Hz, 1H), 4.14 (s, 2H), 3.85 (s, 2H), 3.88-3.82 (m, 1H), 3.80 (s, 1H), 3.80-3.70 (m, 3H), 3.65 (dd,*J*=9.4, 6.9 Hz, 1H), 3.60 (t,*J*=6.5 Hz, 4H), 3.48 (dd,*J*=10.4, 3.9 Hz, 16H), 3.41 (q,*J*=4.8 Hz, 5H), 3.38 (d,*J*=7.0 Hz, 1H), 3.32 (s, 2H), 3.27-3.19 (m, 2H), 3.22 (s, 3H), 2.78 (h,*J*=6.6 Hz, 2H), 2.40 (t,*J*=6.5 Hz, 2H), 2.16 (dtd,*J*=13.4, 8.6, 5.1 Hz, 1H), 1.85 (dqt,*J*=27.7, 13.5, 6.6 Hz, 3H), 1.62 (dp,*J*=13.8, 4.7 Hz, 1H), 1.54 (hpt,*J*=6.6 Hz, 2H), 1.37 (ddt,*J*=23.6, 15.8, 7.0 Hz, 2H), 1.24 (d,*J*=5.7 Hz, 1H), 1.09 (t,*J*=7.0 Hz, 1H), 0.88 (t,*J*=7.3 Hz, 3H).

### 5.2 L4-D2-Ab_{nectin-4}antibody-drug conjugate (ADC5)

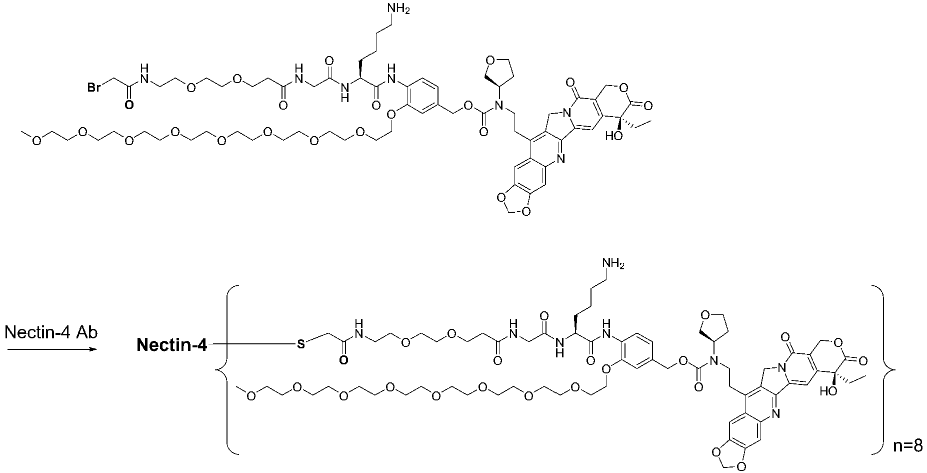

The Nectin-4 antibody solution (10.0 mg/mL, 10 mg, 0.066 mmol) was prepared, and the pH value was adjusted to 7.2 using a 1 M disodium hydrogen phosphate solution. Subsequently, a 0.1 M disodium ethylenediaminetetraacetate solution (25 µL) and a freshly prepared TCEP·HCl solution (10 mM, 0.04 mL) were added sequentially. The mixture was incubated on a rotator at 25°C for 3 h.

Compound **L4-D2** (1.20 mg, 0.80 mmol) was dissolved in 0.12 mL of DMA, and the resulting solution was added to the above reaction system and mixed thoroughly. The mixture was incubated on a rotator at room temperature for 16 h. Upon completion of the reaction, small molecules were removed and the buffer was exchanged into a 20 mM PB solution (pH 6.3) using a NAP-5 gel column (Cytiva) to obtain the antibody-drug conjugate **ADC5** (3.1 mg/mL, 2 mL).

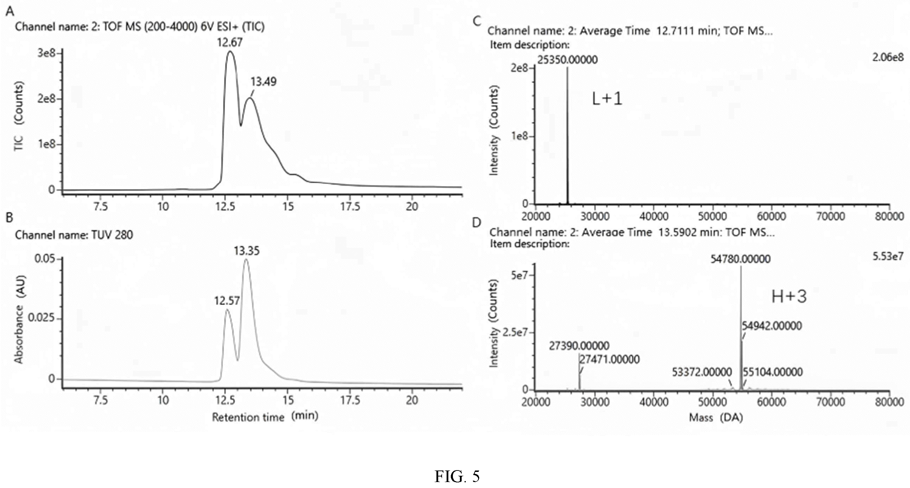

RP-MS calculated: average: n=7.9; MS results showed that the antibody light chain (L) was linked to one **L4-D2** (linker-payload), and the heavy chain (H) was linked to three **L4-D2**(linker-payload) (FIG. 5).

### Test Example: ADC Anti-tumor Activity Assay

### Test Example 1: In vitro inhibitory activity of ADC on tumor cell growth

Human breast cancer cells MDA-MB-468, human bladder transitional cell carcinoma SW-780, human breast cancer cells MCF-7, human prostate cancer cells LNCaP, and human pancreatic adenocarcinoma cells BxPC-3 were cultured to exponential growth phase in DMEM (Cellmax) containing 10% fetal bovine serum (Cellmax), DMEM (Cellmax), MEM (Cellmax), RPMI1640 (Cellmax), and RPMI1640 (Cellmax) media, respectively. The cells were digested with trypsin and centrifuged, then the supernatant was discarded. The cell pellets were diluted with the corresponding culture media to cell concentrations of 6×10⁴ cells/mL, 6×10⁴ cells/mL, 2×10⁴ cells/mL, 3×10⁴ cells/mL and 7×10⁴ cells/mL, respectively. Aliquots of 100 µL cell suspension were added into each well of 96-well cell culture plates. The plates were incubated overnight in an incubator at 37°C with 5% CO₂. On the next day, ADC to be tested was diluted with a culture medium to 12000 nM, 1200 nM, 120 nM, 12 nM, 1.2 nM, 0.12 nM, 0.012 nM, 0.0012 nM, and 0.00012 nM. The diluted ADC was added to the 96-well cell culture plate at the density of 100 µL per well. Three replicate wells were set for each concentration. 100 µL of culture medium was added to each well in negative control (no ADC) and blank control. After addition, the plates were returned to the 37°C, 5% CO₂ incubator and incubated for an additional 6 days. After incubation, the cell culture plates were removed, the culture medium was aspirated using a pipette, 100 µL of culture medium containing 10% CCK-8 was added to each well, and the plates was incubated at 37°C for 3 hours. After incubation, the culture plates were removed, protected from light, and placed in a microplate reader to measure absorbance at a reference wavelength of 630 nm and a measurement wavelength of 450 nm. Based on the absorbance values, IC₅₀ was calculated: using four-parameter regression in GraphPad (Table 1). EV (enfortumab vedotin, Padcev^{®}) was used as a positive control drug.

For the IC₅₀ values, "++++" indicates 50 nM > IC₅₀; "+++" indicates 200 nM > IC₅₀ ≥ 50 nM; "++" indicates 1000 nM > IC₅₀ ≥ 200 nM; "+" indicates IC₅₀> 1 µM.

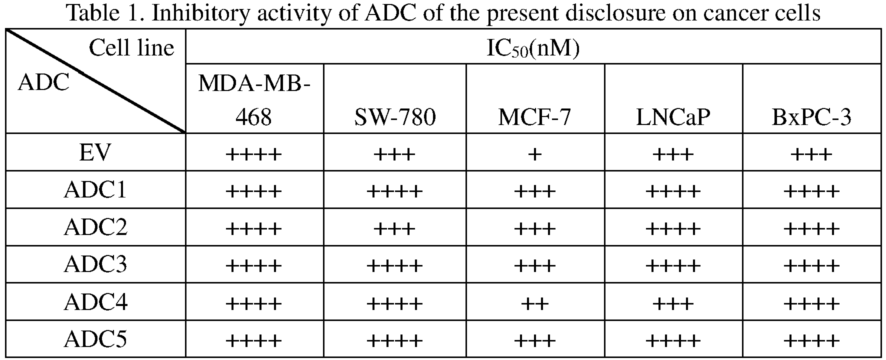

All the ADC compounds provided in the examples of the present disclosure showed good inhibitory activity against cancer cell growth, exhibiting significant anti-cancer activity.

### Test Example 2: In vivo inhibitory activity of ADC on tumor growth

Human breast cancer cells MDA-MB-468, human bladder transitional cell carcinoma SW-780, human lung cancer cells NCI-H292, human bladder cancer cells HT-1376, and human pancreatic adenocarcinoma cells BxPC-3 were cultured in monolayers in vitro. When cell confluence reached 80%-90%, cells were digested with trypsin-EDTA and centrifuged, the supernatant discarded, and the cells were resuspended in PBS. The cell suspension was adjusted to an appropriate concentration. The human breast cancer cells MDA-MB-468, human bladder transitional cell carcinoma SW-780, human lung cancer cells NCI-H292, human bladder cancer cells HT-1376, and human pancreatic adenocarcinoma cells BxPC-3 (2-10×10⁶ cells/0.1mL) were subcutaneously inoculated into BALB/c nude mice. Animals and tumor growth were observed regularly. When the tumor volume reached approximately 100-200 mm³, the mice were randomly grouped based on tumor volume and body weight: a vehicle control group (saline) and ADC administration groups (dissolved in saline), with 6 animals per group. The drugs were administered intravenously once (the first administration time was recorded as Day 0). The long diameter a (mm) and short diameter b (mm) of the tumor and the body weight of the mice were measured with a vernier caliper twice a week. The tumor volume (V) was calculated: according to the following formula: V = 1/2×axb² (mm³), where a and b represent the tumor length and width, respectively. Statistical analysis was based on the tumor volume data at the end of the experiment to calculate the tumor growth inhibition rate, expressed as: 100% * (mean tumor volume of the blank control (vehicle) group - mean tumor volume of the test group)/mean tumor volume of the blank control (vehicle) group.

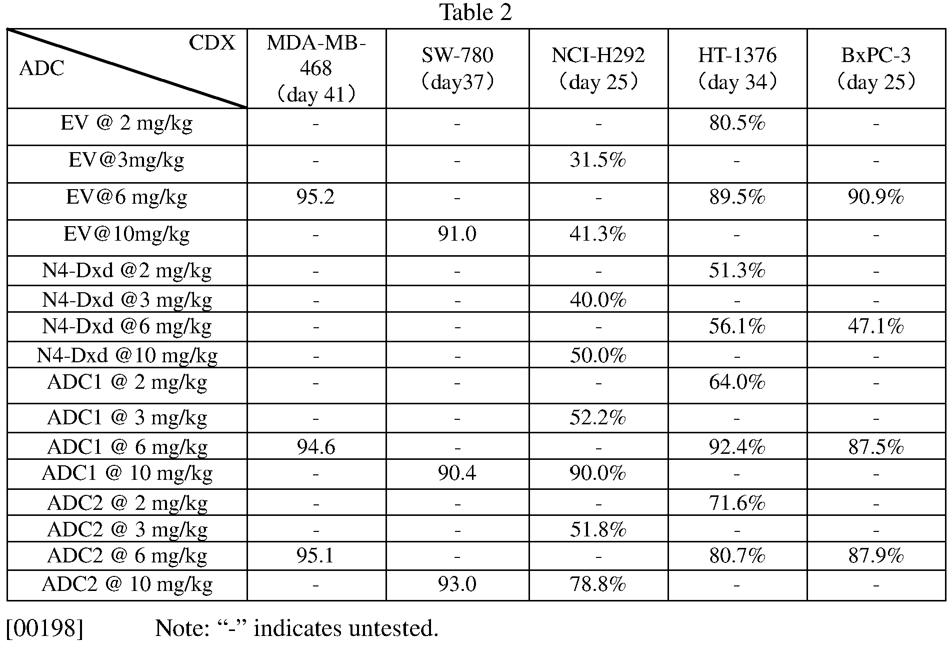

Note: "-" indicates untested

The vehicle group serves as blank control; N4-Dxd represents an ADC formed by conjugating Dxd (GGFG linker) with the anti-Nectin-4 antibody in the examples of the present application;

The structural formula is as follows:

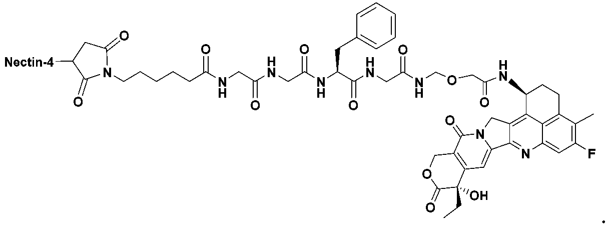

Experimental results: ADC1 and ADC2 at four doses (2 mg/kg, 3 mg/kg, 6 mg/kg, and 10 mg/kg) all showed significant tumor growth inhibitory activity.

The foregoing description of specific exemplary embodiments of the present disclosure is for purposes of illustration and exemplification. These descriptions are not intended to be exhaustive or to limit the invention to the precise forms disclosed. Obviously, many modifications and variations are possible in light of the above teachings. The exemplary embodiments were chosen and described in order to explain certain principles of the invention and their practical application, to thereby enable others skilled in the art to make and utilize various exemplary embodiments of the invention, as well as various alternatives and modifications thereof. It is intended that the scope of the invention be defined by the following claims and their equivalents.

## Claims

1. A compound of Formula (I), a pharmaceutically acceptable salt or a stereoisomer thereof: 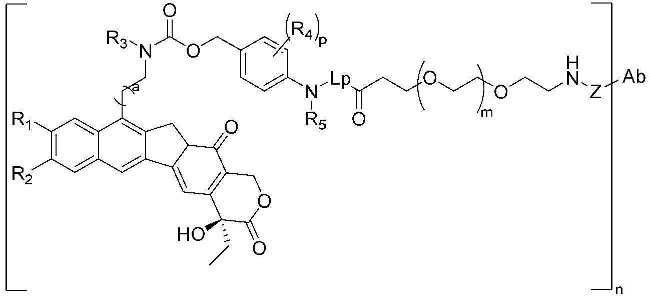 in the formula,
R₁ and R₂ are each independently selected from hydrogen, fluorine, and C₁₋₃ alkyl, or R₁ and R₂ together with the carbon atoms to which they are attached form oxygen-containing heterocyclyl;
R₃ is selected from C₁₋₆ alkyl, C₁₋₆ alkoxy, C₃₋₆ cycloalkyl, or 4- to 8-membered heterocycloalkyl; the C₁₋₆ alkyl and the C₁₋₃ alkoxy are optionally substituted with one or more halogens; the 4- to 8-membered heterocycloalkyl contains 1, 2, or 3 heteroatoms selected from N, O, and S as ring atom;
R₄ is selected from hydrogen or heteroalkyl containing repeating units of -OCH₂CH₂-.
R₅ is selected from hydrogen, C₁₋₆ alkyl, and C₃₋₆ cycloalkyl;
Lp is selected from a peptide residue containing 1-5 amino acids;
m is selected from an integer ranging from 1 to 8;
a and p are selected from 1, 2, or 3;
Ab is an anti-Nectin-4 antibody or an antigen-binding fragment;
Z is a linker capable of conjugating the antibody or the antigen-binding fragment with other part of the compound of Formula (I); $0.5 \leq n \leq 8 .$

2. The compound according to claim 1, wherein R₁ and R₂ are each independently selected from hydrogen, fluorine and methyl, or R₁ and R₂ together with the carbon atoms to which they are attached form 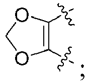
preferably, R₁ is hydrogen, and R₂ is hydrogen;
R₁ is fluorine, and R₂ is fluorine; or,
R₁ is methyl, and R₂ is fluorine.

3. The compound according to claim 1 or 2, wherein R₃ is selected from C₁₋₆ alkyl substituted with 1, 2 or 3 fluorine; or is selected from 4- to 8-membered oxacycloalkyl;
preferably, R₃ is selected from fluoroethyl, difluoroethyl, trifluoroethyl, oxolanyl or oxanyl.

4. The compound according to any one of claims 1 to 3, wherein R₄ is selected from hydrogen, C₁₋₆ alkyl, and -C(O)-NR^{a}R^{b};
R^{a} and R^{b} are each independently selected from C₁₋₆ alkyl;
wherein one or more methylene units in the C₁₋₆alkyl are optionally and independently replaced by -(OCH₂CH₂)q-;
q is selected from an integer ranging from 2 to 10;
preferably, R₄ is selected from hydrogen, 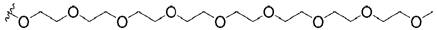 and

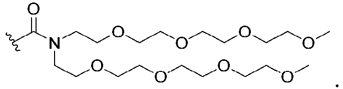

5. The compound according to any one of claims 1-4, wherein L^{p} is selected from -Val-Cit-, -Gly-Lys-, -Gly-Leu-, -Val-Ala-, -Gly-Phe-, -GLy-Gly-Lys-, -Gly-Gly-Phe-, -Gly-Val-Ala-, -Gly-Gly-Val-, -Gly-Leu-Val-, -Gly-Phe-Gly-, or -Gly-Gly-Leu-; preferably, L^{p} is selected from

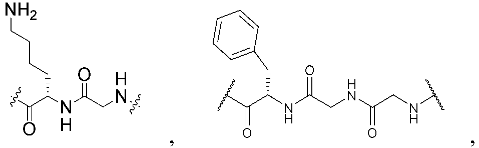

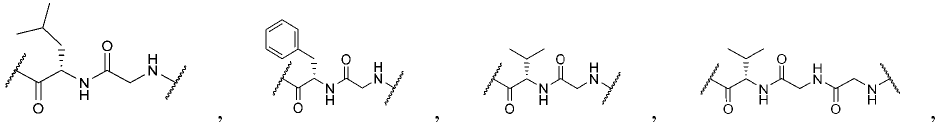

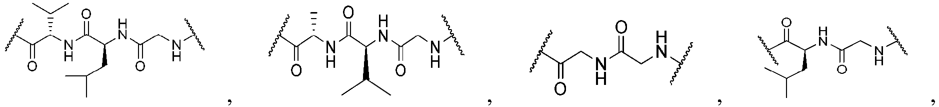

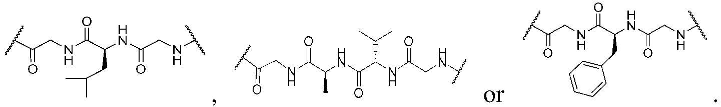

6. The compound according to any one of claims 1 to 5, wherein Z is selected from 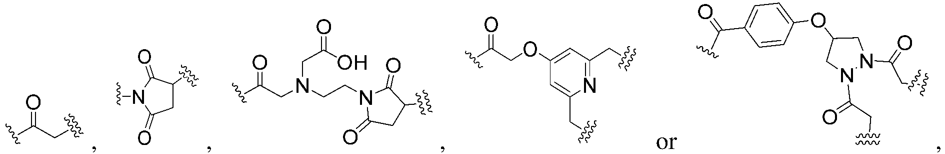 wherein the position indicated by 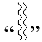 indicates linking to the antibody, and the position indicated by 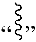 indicates linking to -NH-.

7. The compound according to any one of claims 1 to 6, wherein the heavy chain amino acid sequence of the anti-Nectin-4 antibody is as shown in SEQ ID NO: 1, and the light chain amino acid sequence of the anti-Nectin-4 antibody is as shown in SEQ ID NO: 2.

8. The following compound, a pharmaceutically acceptable salt or a stereoisomer thereof:

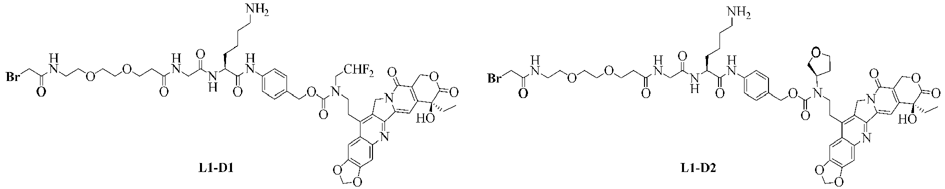

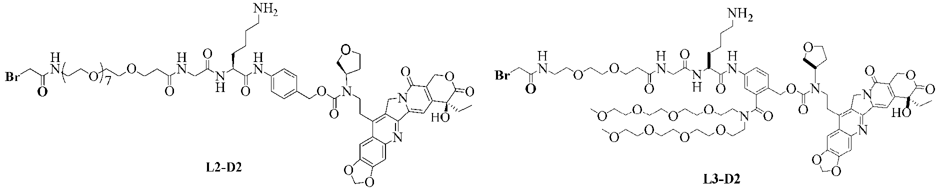

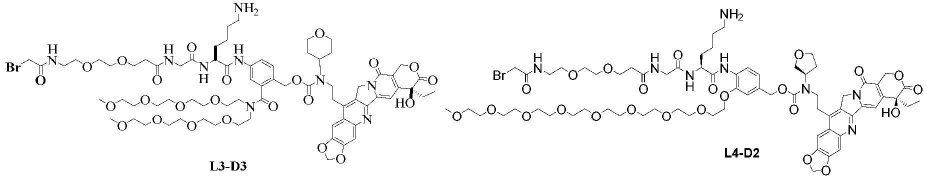

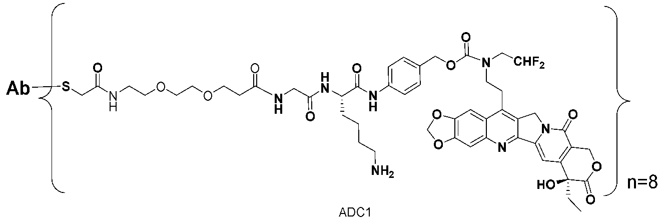

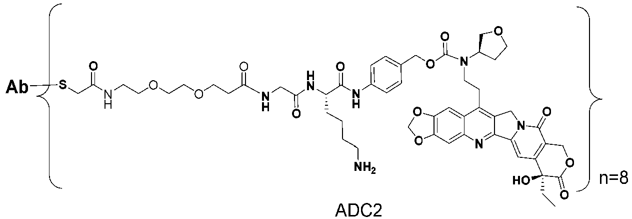

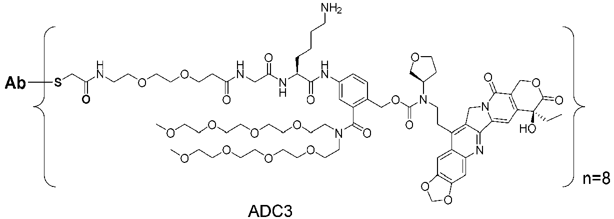

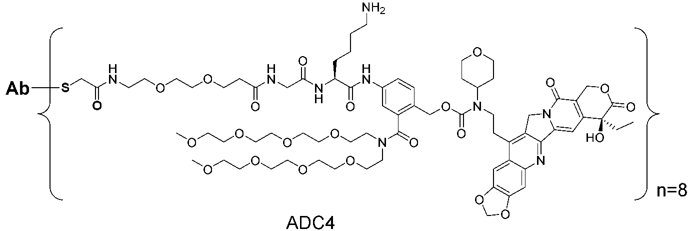

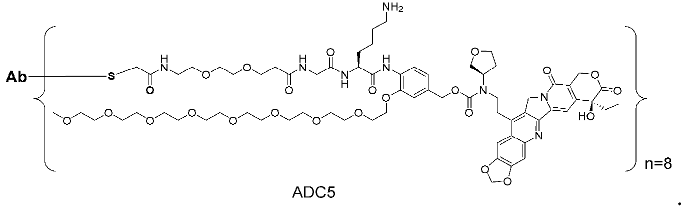

9. A pharmaceutical composition, comprising the compound of Formula (I), the pharmaceutically acceptable salt or the stereoisomer thereof according to any one of claims 1 to 8; and a pharmaceutically acceptable carrier.

10. Use of the compound of Formula (I), the pharmaceutically acceptable salt or the stereoisomer thereof according to any one of claims 1 to 8, or the pharmaceutical composition according to claim 9 in the manufacture of an anti-tumor drug or a drug for treating an autoimmune disease;
preferably, the tumor is selected from solid tumor, more preferably selected from breast cancer, bladder transitional cell carcinoma, prostate cancer, and pancreatic adenocarcinoma.

11. A method of treating cancer, comprising the step of administering to a patient in need thereof the compound of Formula (I), the pharmaceutically acceptable salt or the stereoisomer thereof according to any one of claims 1 to 8, or the pharmaceutical composition according to claim 9;
preferably, the cancer is selected from breast cancer, bladder transitional cell carcinoma, prostate cancer, and pancreatic adenocarcinoma.
